(19) Europäisches Patentamt
European Patent Office
Office européen des brevets

(11) EP 2 556 827 A1

# (12) EUROPEAN PATENT APPLICATION

(43) Date of publication:
**13.02.2013 Bulletin 2013/07**

(21) Application number: **11177210.9**

(22) Date of filing: **11.08.2011**

(51) Int Cl.:
***A61K 31/192*** *(2006.01)* ***A61P 25/16*** *(2006.01)*
***C07C 49/792*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Acadia Pharmaceuticals Inc.**
**San Diego, CA 92121 (US)**

(72) Inventors:
- **Burstein, Ethan S.**
  **San Diego, CA California 92130 (US)**
- **Olsson, Roger**
  **218 38 Bunkeflostrand (SE)**
- **McFarland, Krista**
  **San Marcos, CA California 92069 (US)**

(74) Representative: **Stenbäck, Maria Elisabeth**
**Awapatent AB**
**P.O. Box 5117**
**200 71 Malmö (SE)**

## (54) Treatment of neurodegenerative diseases

(57) The present disclosure relates to compounds to be used in a low dose in treatment of neurodegenerative diseases or disorders. It also relates to methods for treatment of a neurodegenerative diseases or disorders.

EP 2 556 827 A1

## Description

### FIELD

Provided herein are compounds that affect Nurr1 receptors and methods of using such compounds for modulating neurodegenerative conditions.

### BACKGROUND

Nurr1 is a nuclear hormone receptor (NucHR) strongly implicated in the growth, maintenance, and survival of dopaminergic neurons, that represents a very promising therapeutic target for Parkinson's disease (PD). The essential role of Nurr1 in dopaminergic cell development was dramatically demonstrated in mouse gene knockout experiments in which homozygous mice lacking Nurr1 failed to generate midbrain dopaminergic neurons (Zetterstrom et al., 1997). Nurr1 was shown to be directly involved in the regulation of genes coding for aromatic amino acid decarboxylase, tyrosine hydroxylase (TH), and the dopamine transporter (DAT) (Hermanson et al., 2003). In addition, Nurr1 limit inflammatory responses in the CNS and specifically protects dopaminergic neurons from neurotoxicity (Saijo et al., 2009). These observations suggest that Nurr1 play a pathophysiological role in aspects of neurodegenerative diseases ranging from inflammatory responses to dopaminergic nerve function and survival.

For example Nurr1 agonists have great potential as Parkinson's drugs as they enhance TH and DAT expression in primary mensencephalic cultures and exert a beneficial effect on dopaminergic neurons in animal models of PD (Ordentlich et al., 2003; Jankovic et al., 2005; Dubois et al., 2006). However, the molecular basis for the actions of existing ligands is not well defined. Nurr1 may mediate its beneficial effects alone, or more likely in concert with other nuclear hormone receptor partners (Sacchetti et al., 2006; Carpentier et al., 2008). To date, there are a few examples of such ligands available for experimental testing (Shi, 2007).

Nurr1 can form dimers and is known to associate with other NucHRs including PPARγ, GR, FXR and RXR (Sacchetti et al., 2006; Carpentier et al., 2008). It is currently unknown which Nurr1 interaction is therapeutically important in the treatment of PD. However, it is agreed that Nurr1 involvement in dopaminergic neuronal activation and cell survival is important (Shi, 2007). Several of the most potent Nurr1 binding compounds enhance TH and DAT expression in primary mensencephalic cultures and exert a beneficial effect on dopaminergic neurons in animal models ofPD (Jankovic et al., 2005).

Accordingly, there is a need for compounds, such as Nurr1 agonists, or compounds that induce activation of Nurr1 indirectly through Nurr1 binding partners that are neuroprotective via activity at the Nurr1 receptor in the central nervous system, both as pharmacological tools and as therapeutic agents.

### SUMMARY

Some embodiments relate to a compound of formula (I)

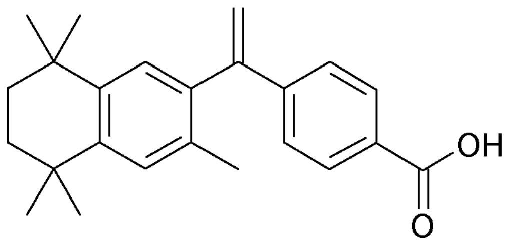

(I)

or a pharmaceutically acceptable salt, solvate, polymorph or hydrate thereof, for use in the treatment of a neurodegenerative disease or disorder wherein said compound is to be administered in a low dose.

Some embodiments relate to the use of a compound of formula (I)

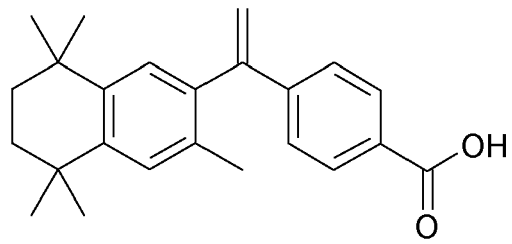

(I)

or a pharmaceutically acceptable salt, solvate, polymorph or hydrate thereof, for the manufacture of a pharmaceutical composition for use in the treatment of a neurodegenerative disease or disorder wherein said compound is to be administered in a low dose.

Some embodiments relate to a method for the treatment of a neurodegenerative disease or disorder, comprising the administration to a patient having a neurodegenerative disease or disorder an effective amount of the compound of formula (I)

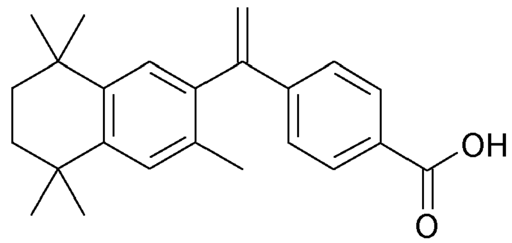

(I)

or a pharmaceutically acceptable salt, solvate, polymorph or hydrate thereof, wherein the compound is administered to the patient at a low dose.

Some embodiments relate to a method for the regeneration of the function of neurons in a patient having a neurodegenerative disease or disorder, comprising the administration to the patient having a neurodegenerative disease or disorder an effective amount of the compound of formula (I)

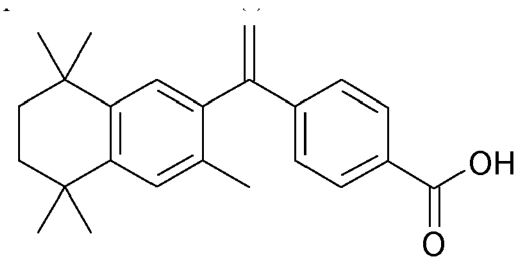

(I)

or a pharmaceutically acceptable salt, solvate, polymorph or hydrate thereof.

Some embodiments relate to a method for the protection of neurons in a patient having a neurodegenerative disease or disorder, comprising the administration to the patient having a neurodegenerative disease or disorder an effective amount of the compound of formula (I)

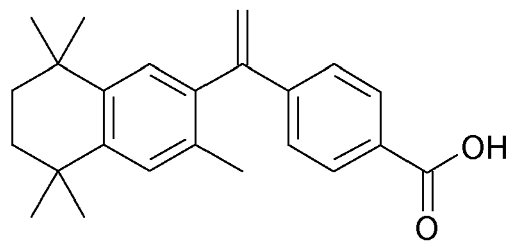

(I)

or a pharmaceutically acceptable salt, solvate, polymorph or hydrate thereof wherein the compound is administered to the patient at a dose low dose.

Some embodiments provide a pharmaceutical composition, comprising an effective amount ofbexarotene (the compound of formula (I)) or a pharmaceutically acceptable salt, solvate, polymorph or hydrate thereof.

## DETAILED DESCRIPTION OF EMBODIMENTS

### Definitions

Unless defined otherwise, all technical and scientific terms used herein have the same meaning as is commonly understood by one of ordinary skill in the art. All patents, applications, published applications and other publications referenced herein are incorporated by reference in their entirety. In the event that there is a plurality of definitions for a term herein, those in this section prevail unless stated otherwise

The term "neurodegenerative disease or disorder" as used herein refers to a disease or disorder selected from the group consisting of Parkinson’s disease, Alzheimer’s disease, Huntington’s disease, frontotemporal lobar degeneration associated with protein TDP-43 (FTLD-TDP, Dementia with Lewy bodies (DLB), vascular dementia, Amyotrophic lateral sclerosis (ALS), and other dementias resulting in impaired cognitive function (cognition) due to changes in the brain caused by ageing, disease or trauma; or spinal cord injury. Such neurodegenerative diseases or disorders may be associated with a Nurr1 receptor.

The term "neuroprotection" as used herein refers to the prevention of further loss of neuronal cells, or loss of neuronal function as a result of exposure to a neurotoxin or resulting from a neurodegenerative disease or disorder. As used herein, the term "neuroprotection" is synonymous with "protection of neurons".

As used herein, promotion of neuronal survival is considered equivalent to neuroprotection

The term "regeneration" as used herein refers to enabling an increase in the activity of an injured or disabled cell, or a cell having below normal activity relative to the natural activity of a corresponding healthy cell. Such a cell may be a neuron. In some embodiments provided herein, "regeneration" refers to the regeneration of neurons in a patient having a neurodegenerative disease or disorder.

Thus, in some embodiments "neuroregeneration" refers to the regeneration of neurons in a patient having a neurodegenerative disease or disorder. In some embodiments, "neuroregeneration refers to the process of reversing either the loss of neuronal cells, or the loss of neuronal function occurring as a result of exposure to a neurotoxin or resulting from a neurodegenerative disease.

Neurorestoration shall be defined to be equivalent to neuroregeneration. The term "neuronal function" as used herein refers to the capability of a neuron to synthesize, store, release, transport and respond to a neurotransmitter. Thus, changes in expression or integrity of certain components of neurons, including but not limited to receptors transporters, vesicles, cell bodies, axons or dendrites may affect neuronal function.

Neurotransmitters shall be defined as diffusible molecules released by neurons that either stimulate or inhibit neuronal activity.

The expression "low dose" as used herein refers to a dose of a compound or drug, e.g. bexarotene, not greater than 75 mg per day for a human patient. To obtain the desired effect of the compound or drug, at least when bexarotene is used, the dose shall be at least 0.05 mg per day for a human patient. In some embodiments, the "low dose" may thus be a dose of about 0.05 mg to about 75 mg per day. The low dose may be give as one single daily dose or as a series of several doses or as a continuous infusion with a total daily dose of about 0.05 mg to about 75 mg. It is also possible to to give the total low daily dose through at least two different routes of administration. Without being bound by any particular theory, it may be possible to use a low dose of bexarotene as provided herein based on the surprising finding that bexarotene is more than 10-fold more potent in stimulating Nurr-1-RXR heterodimers than RXR-RXR homodimers. Hence, for clinical applications that depend on Nurr-1 stimulation, bexarotene can be used in much lower and much more tolerated doses than are used in anti-cancer therapy. This is supported by studies in an animal model ofPD that show neuroprotective and neuroregenerative effects of very low doses of bexarotene, as shown further below. Bexarotene is a RXR agonist that acts through the homodimer RXR-RXR to produce clinically used anti-cancer effects.

As used herein, "pharmaceutically acceptable salt" refers to a salt of a compound that does not per se abrogate the biological activity and properties of the compound. Pharmaceutical salts can be obtained by reaction of a compound disclosed herein with a base. Base-formed salts include, without limitation, ammonium salt ($NH_4^+$); alkali metal, such as, without limitation, sodium or potassium, salts; alkaline earth, such as, without limitation, calcium or magnesium, salts; salts of organic bases such as, without limitation, dicyclohexylamine, N-methyl-D-glucamine, tris(hydroxymethyl)methylamine; and salts with the amino group of amino acids such as, without limitation, arginine and lysine.

Pharmaceutically acceptable solvates and hydrates are complexes of a compound with one or more solvent of water molecules, or 1 to about 100, or 1 to about 10, or one to about 2, 3 or 4, solvent or water molecules.

As used herein, to "modulate" the activity of a receptor means either to activate it, i.e., to increase its cellular

function over the base level measured in the particular environment in which it is found, or deactivate it, i.e., decrease its cellular function to less than the measured base level in the environment in which it is found and/or render it unable to perform its cellular function at all, even in the presence of a natural binding partner. A natural binding partner is an endogenous molecule that is an agonist for the receptor.

An "agonist" is defined as a compound that increases the basal activity of a receptor (i.e. signal transduction mediated by the receptor).

As used herein, "partial agonist" refers to a compound that has an affinity for a receptor but, unlike an agonist, when bound to the receptor it elicits only a fractional degree of the pharmacological response normally associated with the receptor even if a large number of receptors are occupied by the compound.

An "inverse agonist" is defined as a compound, which reduces, or suppresses the basal activity of a receptor, such that the compound is not technically an antagonist but, rather, is an agonist with negative intrinsic activity.

As used herein, "antagonist" refers to a compound that binds to a receptor to form a complex that does not give rise to any response, as if the receptor was unoccupied. An antagonist attenuates the action of an agonist on a receptor. An antagonist may bind reversibly or irreversibly, effectively eliminating the activity of the receptor permanently or at least until the antagonist is metabolized or dissociates or is otherwise removed by a physical or biological process.

As used herein, a "subject" refers to an animal that is the object of treatment, observation or experiment. "Animal" includes cold- and warm-blooded vertebrates and invertebrates such as fish, shellfish, reptiles and, in particular, mammals. "Mammal" includes, without limitation, mice; rats; rabbits; guinea pigs; dogs; cats; sheep; goats; cows; horses; primates, such as monkeys, chimpanzees, and apes, and, in particular, humans.

As used herein, a "patient" refers to a subject that is being treated by a medical professional such as an M.D. or a D.V.M. to attempt to cure, or at least ameliorate the effects of, a particular disease or disorder or to prevent the disease or disorder from occurring in the first place.

As used herein, a "carrier" refers to a compound that facilitates the incorporation of a compound into cells or tissues. For example, without limitation, dimethyl sulfoxide (DMSO) is a commonly utilized carrier that facilitates the uptake of many organic compounds into cells or tissues of a subject.

As used herein, a "diluent" refers to an ingredient in a pharmaceutical composition that lacks pharmacological activity but may be pharmaceutically necessary or desirable. For example, a diluent may be used to increase the bulk of a potent drug whose mass is too small for manufacture or administration. It may also be a liquid for the dissolution of a drug to be administered by injection, ingestion or inhalation. A common form of diluent in the art is a buffered aqueous solution such as, without limitation, phosphate buffered saline that mimics the composition of human blood.

As used herein, an "excipient" refers to an inert substance that is added to a pharmaceutical composition to provide, without limitation, bulk, consistency, stability, binding ability, lubrication, disintegrating ability etc., to the composition. A "diluent" is a type of excipient.

A "receptor" is intended to include any molecule present inside or on the surface of a cell that may affect cellular physiology when it is inhibited or stimulated by a ligand. Typically, a receptor comprises an extracellular domain with ligand-binding properties, a transmembrane domain that anchors the receptor in the cell membrane, and a cytoplasmic domain that generates a cellular signal in response to ligand binding ("signal transduction"). A receptor also includes any molecule having the characteristic structure of a receptor, but with no identifiable ligand. In addition, a receptor includes a truncated, modified, mutated receptor, or any molecule comprising partial or all of the sequences of a receptor.

"Ligand" is intended to include any substance that interacts with a receptor.

The "Nurr1 receptor" is defined as a receptor having an activity corresponding to the activity of the Nurr1 receptor subtype characterized through molecular cloning and pharmacology.

As used herein, "coadministration" of pharmacologically active compounds refers to the delivery of two or more separate chemical entities, whether in vitro or in vivo. Coadministration means the simultaneous delivery of separate agents; the simultaneous delivery of a mixture of agents; as well as the delivery of one agent followed by delivery of a second agent or additional agents. Agents that are coadministered are typically intended to work in conjunction with each other.

The term "an effective amount" as used herein means an amount of active compound or pharmaceutical agent that elicits the biological or medicinal response in a tissue, system, animal or human that is being sought by a researcher, veterinarian, medical doctor or other clinician, which includes alleviation or palliation of the symptoms of the disease being treated.

Compounds

The compound as provided herein is bexarotene, the compound according to formula I (also known under the tradename Targretin and as LGD1069),

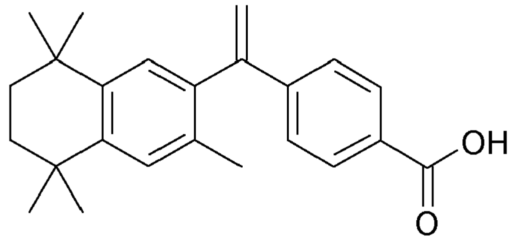

(I)

or a pharmaceutically acceptable salt, solvate, polymorph or hydrate thereof.

In some embodiments, bexarotene or a pharmaceutically acceptable salt, solvate, polymorph or hydrate thereof is coadministered with at least one other pharmacologically active compound.

As disclosed herein bexarotene or a pharmaceutically acceptable salt, solvate, polymorph or hydrate thereof or a pharmaceutical composition comprising any of these is to be administered in a low dose in any known or/and conventional administration route. Examples of suitable routes of administration include oral, rectal, transmucosal (including sublingual and buccal), topical, transdermal or intestinal administration; parenteral delivery, including intramuscular, subcutaneous, intravenous, intramedullary injections, as well as intrathecal, direct intracerebroventricular injection, direct injections to the human brain, direct intraventricular, intraperitoneal, intranasal, or intraocular injections. The compounds can also be administered in sustained or controlled release dosage forms, including nanoparticles, depot injections, osmotic pumps, electronic pumps, pills, transdermal (including electrotransport) patches, and the like, for prolonged and/or timed, pulsed administration at a predetermined rate. Sustained or controlled release dosage forms may be used to increase CNS exposure and minimize systemic exposure. It is also possible to combine at least two different routes of administration.

In some embodiments the compound is to be administered to a patient having a neurodegenerative disease or disorder in a dose of from 0.05 mg up to, and including, 75 mg per day.

In some embodiments the compound is to be administered to a patient having a neurodegenerative disease or disorder in a dose of up to, and including, 70 mg per day. Some of these embodiments may relate to oral administration.

In some embodiments the compound is to be administered to a patient having a neurodegenerative disease or disorder in a dose of up to, and including, 65 mg per day.

In some embodiments the compound is to be administered to a patient having a neurodegenerative disease or disorder in a dose of up to, and including, 50 mg per day.

In some embodiments the compound is to be administered to a patient having a neurodegenerative disease or disorder in a dose of up to, and including, 20 mg per day. Some of these embodiments may relate to intracerebroventricular administration.

In some embodiments the compound is to be administered to a patient having a neurodegenerative disease or disorder in a dose of up to, and including, 15 mg per day. Some of these embodiments may relate to intracerebroventricular administration.

The lower limit of the dose range may be 0.05 mg per day, as indicated further above.

In some embodiments, the lower limit of the dose range may be 0.1 mg per day, with the upper limit according to any of the alternative embodiments given above.

In some embodiments, the lower limit of the dose range may be 0.5 mg per day, with the upper limit according to any of the alternative embodiments given above.

In some embodiments, the lower limit of the dose range may be 1 mg per day, with the upper limit according to any of the alternative embodiments given above.

In some embodiments, the lower limit of the dose range may be 5 mg per day, with the upper limit according to any of the alternative embodiments given above. Some of these embodiments may relate to oral administration.

In some embodiments the dose range may be selected from the group consisting of:

from 0.05 mg up to, and including, 75 mg per day,
from 0.05 mg up to, and including, 70 mg per day,
from 0.05 mg up to, and including, 65 mg per day,
from 0.05 mg up to, and including, 50 mg per day,
from 0.05 mg up to, and including, 20 mg per day,
from 0.05 mg up to, and including, 15 mg per day,
from 0.1 mg up to, and including, 75 mg per day,
from 0.1 mg up to, and including, 70 mg per day,
from 0.1 mg up to, and including, 65 mg per day,

from 0.1 mg up to, and including, 50 mg per day,
from 0.1 mg up to, and including, 20 mg per day,
from 0.1 mg up to, and including, 15 mg per day,
from 0.5 mg up to, and including, 75 mg per day,
from 0.5 mg up to, and including, 70 mg per day,
from 0.5 mg up to, and including, 65 mg per day,
from 0.5 mg up to, and including, 50 mg per day,
from 0.5 mg up to, and including, 20 mg per day,
from 0.5 mg up to, and including, 15 mg per day,
from 1 mg up to, and including, 75 mg per day,
from 1 mg up to, and including, 70 mg per day,
from 1 mg up to, and including, 65 mg per day,
from 1 mg up to, and including, 50 mg per day,
from 1 mg up to, and including, 20 mg per day,
from 1 mg up to, and including, 15 mg per day,
from 5 mg up to, and including, 75 mg per day,
from 5 mg up to, and including, 70 mg per day,
from 5 mg up to, and including, 65 mg per day,
from 5 mg up to, and including, 50 mg per day,
from 5 mg up to, and including, 20 mg per day, and
from 5 mg up to, and including, 15 mg per day.

In some embodiments the compound may be administered non-orally. Non-oral administration means that the treatment may be safer and more effective compared to oral administration may be more easily tolerated by the subject since it is possible to use a lower total dose of bexarotene or the pharmaceutically acceptable salt, solvate, polymorph or hydrate thereof, that the effects on liver function are reduced because the maximum concentrations of drug the liver is exposed to are reduced, and that the distribution ofbexarotene in the body is altered such that a greater proportion of the administered dose reaches the brain compared with the periphery, thereby reducing many side-effects earlier associated with bexarotene.

In some embodiments the compound may be administered intracerebroventricularly (i.c.v.). I.c.v. administration means that the treatment may be safer and more effective compared to oral administration since it is possible to use a much lower total dose ofbexarotene or the pharmaceutically acceptable salt, solvate, polymorph or hydrate thereof and that the distribution ofbexarotene in the body is altered such that the vast majority of the administered dose is in the brain but very little gets into the periphery, thereby avoiding many side-effects earlier associated with bexarotene. This also improves the efficacy ofbexarotene, since high concentrations are delivered into the brain with minimal concentrations in the periphery.

Some embodiments wherein intracerebroventricular administration may be preferred may relate to treatment of Parkinson's disease.

Other embodiments wherein intracerebroventricular administration may be preferred may relate to treatment of Alzheimer's disease, Huntington's disease, frontotemporal lobar degeneration associated with protein TDP-43 (FTLD-TDP), Dementia with Lewy bodies (DLB), vascular dementia and/or Amyotrophic lateral sclerosis

(ALS).

In some embodiment subcutaneous administration may be preferred. Some of these embodiments may relate to treatment of Parkinson's disease.

Other embodiments wherein subcutaneous administration may be preferred may relate to treatment of Alzheimer's disease, Huntington's disease, frontotemporal lobar degeneration associated with protein TDP-43 (FTLD-TDP), Dementia with Lewy bodies (DLB), vascular dementia and/or Amyotrophic lateral sclerosis (ALS). In some embodiment topical or transdermal administration may be preferred. Some of these embodiments may relate to treatment of Parkinson's disease.

Other embodiments wherein topical or transdermal administration administration may be preferred may relate to treatment of Alzheimer's disease, Huntington's disease, frontotemporal lobar degeneration associated with protein TDP-43 (FTLD-TDP), Dementia with Lewy bodies (DLB), vascular dementia and/or Amyotrophic lateral sclerosis (ALS).

In the context of the present disclosure it has been shown that it is possible to administer bexarotene or a pharmaceutically acceptable salt, solvate, polymorph or hydrate thereof or a pharmaceutical composition comprising any of these in a low dose, as defined above, thereby minimizing the deleterious side effects but still obtaining the desired therapeutic effect.

Such deleterious side effects that may be decreased or minimized according to the present disclosure include, but are not limited to i.a. hyperlipidaemia, acute pancreatitis, liver function test (LFT) abnormalities and in particular LFT elevations, thyroid function test alterations and most often elevations in serum triglycerides and serum cholesterol, reductions in thyroid hormone (total thyroxine, $T_4$) and thyroid-stimulating hormone (TSH), leucopenia, anaemia, lens opacities, hypoglycaemia in patients with diabetes mellitus, bleeding, hemorrhage, and coagulopathy, dyspnea, nausea, neuropathic pain, edema, anorexia, asthenia, fatigue, leucopenia, pancreatitis and dehydration and photosensitivity.

In some embodiments the negative side effect to be minimized is hyperlipidaemia.

In some embodiments the negative side effect to be minimized is hypertriglyceradaemia.

In some embodiments the negative side effect to be minimized is hypercholesterolaemia.

In some embodiments the negative side effect to be minimized is the reduction of $T_4$ levels.

In some embodiments the negative side effect to be minimized is the reduction of TSH levels.

When administered to a subject or a patient, bexarotene or a pharmaceutically acceptable salt, solvate, polymorph or hydrate thereof or a pharmaceutical composition comprising any of these may lead to regeneration of the function of dopaminergic neurons.

According to the present disclosure it may thus be possible to restore function to dopaminergic neurons that have lost function due to a neurodegenerative disorder or a neurodegenerative condition. Possible ways of measuring restoration of the function of dopaminergic neurons in humans afflicted with a neurodegenerative disorder or a neurodegenerative condition include, but are not limited to using PET (positron emission tomography) to measure dopamine turnover, or DAT (dopamine transporter) activity, or neuroinflammatory markers.

In some embodiments the dopaminergic neurons have lost their function partially due to Parkinson's disease. The fact that the function of dopaminergic neurons may be regenerated means that it may be possible to reverse the progression of the disease and reduce symptoms in patients with Parkinson's disease. This is not possible with compounds that only slow down the progression of the disease. Possible ways of measuring the effect on neurodegeneration or neuroregeneration include, but are not limited tousing PET (positron emission tomography) to measure dopamine turnover, or DAT (dopamine transporter) activity, or neuroinflammatory markers.

Bexarotene or a pharmaceutically acceptable salt, solvate, polymorph or hydrate thereof or a pharmaceutical composition comprising any of these may therefore be used in treatment of a disease or disorder that benefits from regeneration of dopaminergic neurons.

Such diseases or disorders that benefits from regeneration of dopaminergic neurons may be diseases or disorders associated with a Nurr1 receptor.

In some embodiments the compound as provided herein or pharmaceutically acceptable salt, solvate, polymorph or hydrate thereof leads to an increased activity of a Nurr1 receptor upon administration to the subject.

In some embodiments the activity of the Nurr1 receptor is a signaling activity of a receptor complex including the Nurr1 receptor.

In some embodiments the activity of the Nurr1 receptor is associated with Nurr1 receptor activation.

In some embodiments the Nurr1 receptor is located in the subject's central nervous system.

The compound may form part of a pharmaceutical composition. The term "pharmaceutical composition" refers to a mixture of a compound disclosed herein with other chemical components, such as diluents or carriers. The introduction of the compound into a pharmaceutical composition facilitates administration of the compound to an organism. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" defines chemical compounds diluted in water that will dissolve the compound of interest as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

The term "physiologically acceptable" defines a carrier or diluent that does not abrogate the biological activity and properties of the compound.

The pharmaceutical compositions described herein can be administered to a human patient *per se,* or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, PA, 18th edition, 1990.

The pharmaceutical compositions ofbexarotene may be manufactured in a manner that is itself known, e.g., by means of conventional mixing, dissolving, granulating, dragee-making, levigating, emulsifying, encapsulating, entrapping or tabletting processes.

Pharmaceutical compositions ofbexarotene for use as described herein may be formulated in conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as suitable and as understood in the art; e.g., in Remington's Pharmaceutical Sciences, above.

Injectables can be prepared in conventional forms, either as liquid solutions or suspensions, solid forms suitable for solution or suspension in liquid prior to injection, or as emulsions. Suitable excipients are, for example, water, saline, dextrose, mannitol, lactose, lecithin, albumin, sodium glutamate, cysteine hydrochloride, and the like. In addition, if desired, the injectable pharmaceutical compositions may contain minor amounts of nontoxic auxiliary substances, such as wetting agents, pH buffering agents, and the like. Physiologically compatible buffers include, but are not limited to, Hanks's solution, Ringer's solution, or physiological saline buffer. If desired, absorption enhancing preparations (for example, liposomes), may be utilized.

For transmucosal administration, penetrants appropriate to the barrier to be permeated may be used in the formulation.

For transdermal administration, the composition may be formulated as a gel.

Pharmaceutical formulations for parenteral administration, e.g., by bolus injection or continuous infusion, include aqueous solutions of the active compounds in water-soluble form. Additionally, suspensions of the active compounds may be prepared as appropriate oily injection suspensions. Suitable lipophilic solvents or vehicles include fatty oils such as sesame oil, or other organic oils such as soybean, grapefruit or almond oils, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions. Formulations for injection may be presented in unit dosage form, *e.g.*, in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

For oral administration, bexarotene can be formulated readily by combining the active compounds with pharmaceutically acceptable carriers well known in the art. Such carriers enable the compounds disclosed herein to be formulated as tablets, pills, dragees, capsules, liquids, gels, syrups, slurries, suspensions and the like, for oral ingestion by a patient to be treated. Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

Buccal administration refers to placing a tablet between the teeth and the mucous membranes of the cheek any composition suitable therefor is thus contemplated. The compositions may for example take the form of tablets or lozenges formulated in conventional manner.

For administration by inhalation, bexarotene, for use as described herein, are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the

case of a pressurized aerosol the dosage unit may be determined by providing a valve to deliver a metered amount. Capsules and cartridges of, *e.g.,* gelatin for use in an inhaler or insufflator may be formulated containing a powder mix of the compound and a suitable powder base such as lactose or starch.

Further disclosed herein are various pharmaceutical compositions well known in the pharmaceutical art for uses that include intraocular, intranasal, and intraauricular delivery. Suitable penetrants for these uses are generally known in the art. Pharmaceutical compositions for intraocular delivery include aqueous ophthalmic solutions of the active compounds in water-soluble form, such as eyedrops, or in gellan gum (Shedden et al., Clin. Ther., 23(3):440-50 (2001)) or hydrogels (Mayer et al., Ophthalmologica, 210(2):101-3 (1996)); ophthalmic ointments; ophthalmic suspensions, such as microparticulates, drug-containing small polymeric particles that are suspended in a liquid carrier medium (Joshi, A., J. Ocul. Pharmacol., 10(1):29-45 (1994)), lipid-soluble formulations (Alm et al., Prog. Clin. Biol. Res., 312:447-58 (1989)), and microspheres (Mordenti, Toxicol. Sci., 52(1):101-6 (1999)); and ocular inserts. All of the above-mentioned references, are incorporated herein by reference in their entireties. Such suitable pharmaceutical formulations are most often and preferably formulated to be sterile, isotonic and buffered for stability and comfort. Pharmaceutical compositions for intranasal delivery may also include drops and sprays often prepared to simulate in many respects nasal secretions to ensure maintenance of normal ciliary action. As disclosed in Remington's Pharmaceutical Sciences, 18th Ed., Mack Publishing Co., Easton, PA (1990), which is incorporated herein by reference in its entirety, and well-known to those skilled in the art, suitable formulations are most often and preferably isotonic, slightly buffered to maintain a pH of 5.5 to 6.5, and most often and preferably include antimicrobial preservatives and appropriate drug stabilizers. Pharmaceutical formulations for intraauricular delivery include suspensions and ointments for topical application in the ear. Common solvents for such aural formulations include glycerin and water.

Bexarotene may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For hydrophobic compounds, a suitable pharmaceutical carrier may be a co-solvent system comprising benzyl alcohol, a nonpolar surfactant, a water-miscible organic polymer, and an aqueous phase. A common cosolvent system used is the VPD co-solvent system, which is a solution of 3% w/v benzyl alcohol, 8% w/v of the nonpolar surfactant Polysorbate 80™, and 65% w/v polyethylene glycol 300, made up to volume in absolute ethanol. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of POLYSORBATE 80™; the fraction size of polyethylene glycol may be varied; other biocompatible polymers may replace polyethylene glycol, *e.g.*, polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

Agents intended to be administered intracellularly may be administered using techniques well known to those of ordinary skill in the art. For example, such agents may be encapsulated into liposomes. All molecules present in an aqueous solution at the time of liposome formation are incorporated into the aqueous interior. The liposomal contents are both protected from the external micro-environment and, because liposomes fuse with cell membranes, are efficiently delivered into the cell cytoplasm. The liposome may be coated with a tissue-specific antibody. The liposomes will be targeted to and taken up selectively by the desired organ. Alternatively, small hydrophobic organic molecules may be directly administered intracellularly.

Additional therapeutic or diagnostic agents may be incorporated into the pharmaceutical compositions. Alternatively or additionally, pharmaceutical compositions may be combined with other compositions that contain other therapeutic or diagnostic agents.

Methods of Administration

Bexarotene may be administered to the patient by any suitable means. Nonlimiting examples of methods of

administration include, among others, (a) administration though oral pathways, which administration includes administration in capsule, tablet, granule, spray, syrup, or other such forms; (b) administration through non-oral pathways such as rectal, vaginal, intraurethral, intraocular, intranasal, intracerebroventricular or intraauricular, which administration includes administration as an aqueous suspension, an oily preparation or the like or as a drip, spray, suppository, salve, ointment or the like; (c) administration via injection, subcutaneously, intraperitoneally, intravenously, intramuscularly, transdermally, intraorbitally, intracapsularly, intraspinally, intrasternally, intracranially, intracerebroventricularly or the like, including infusion pump delivery; (d) administration locally such as by injection directly in the renal or cardiac area, e.g., by depot implantation; (e) administration topically; as deemed appropriate by those of skill in the art for bringing the compound disclosed herein into contact with living tissue as well as f) administration as aerosols via inhalation.

Pharmaceutical compositions ofbexarotene suitable for administration include compositions where the active ingredients are contained in an amount effective to achieve its intended purpose. However, as indicated above, the compound is to be administered in a low dose. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

As will be readily apparent to one skilled in the art, the useful *in vivo* dosage to be administered and the particular mode of administration will vary depending upon the age, weight and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential products are commenced at higher dosage levels, with dosage being decreased until the desired effect is no longer achieved or adverse side effects disappear. Alternatively dosages may be based and calculated upon the surface area of the patient, as understood by those of skill in the art.

The exact formulation, route of administration and dosage for the pharmaceutical compositions disclosed herein can be chosen by the individual physician in view of the patient's condition. (See *e.g.,* Fingl et al. 1975, in "The Pharmacological Basis of Therapeutics", which is hereby incorporated herein by reference in its entirety, with particular reference to Ch. 1, p. 1). The dosage may be a single one or a series of two or more given in the course of one or more days, as is needed by the patient.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

In some embodiments, the compounds will be administered for a period of continuous therapy, for example for a week or more, or for months or years.

The amount of composition administered may be dependent on the subject being treated, on the subject's weight, the severity of the affliction, the manner of administration and the judgment of the prescribing physician.

The compositions ofbexarotene may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions comprising a compound disclosed herein formulated in a compatible pharmaceutical carrier may also be prepared, placed in an appropriate container, and labeled for treatment of an indicated condition.

Further details are provided in the following examples, which are not in any way intended to limit the scope of the invention as claimed.

BRIEF DESCRIPTION OF THE DRAWINGS

In the following examples reference is made to the appended drawings which illustrate the following.

Figure 1: discloses BRET constructs, where receptors are drawn with the amino-terminus on the left. Vertical lines denotes Green Fluorescent Protein (GFP2). A grid denotes Renilla luciferase (Rluc).

Figure 2 discloses pharmacological profiling in BRET. Pairs of receptors, one tagged with Luc, one with GFP, were co-expressed, except for receptors labeled DT which were fused to both tags. BRET assays were performed using the indicated concentrations of ligands. Information about each compound is found in Table 1.

Figure 3 illustrates bexarotene activity in NBRE enhancer driven luciferase reporter assays.

Figure 4 illustrates neuroprotective effects of bexarotene in 6-OHDA treated rats.

Figure 5 displays the motor performance of sham (all treatments combined) and 6-hydroxydopamine animals treated with vehicle (Veh), or bexarotene 72 hours following 60HDA infusion (bex(72)). Panels A and B show the start latency and time required to traverse the challenging beam, respectively. Panels C and D show trial time and rpm achieved on the rotorod, respectively. Panel E shows distance traveled during a 15 min spontaneous locomotor session. For each of these measures of motoric ability, 60HDA treatment statistically impaired performance. In all cases, lesioned animals treated with bexarotene (beginning 72 after lesion) were not impaired relative to Sham controls. Data were analyzed using one-way ANOVAs, followed by Bonferroni's multiple comparison post hoc analyses. * indicates a significant difference from sham treated animals, $p<0.05$. + indicates a significant difference from vehicle/6OHDA, $p<0.05$. N=3-5 animals per group.

Figure 6 shows tyrosine hydroxylase immunofluorescence in the SN following sham- or 60HDA-treatment. 60HDA resulted in reduced cell counts in the SN (Panel A), reduced mean cell size (Panel B), reduced mean pixel intensity of immunofluorescent pixels (Panel C) and reduced percentage of the image that was immunopositive (Panel D). Treatment with bexarotene beginning 72 hours after 60HDA lesion significantly improved cell counts, cell size and mean pixel intensity compared to vehicle treated subjects. Data were analyzed with one-way ANOVAs followed by Bonferroni's post hoc comparisons. * indicates a significant difference from Sham, $p<0.05$; + indicates a significant difference from vehicle/60HDA, $p<0.05$.

Figure 7 shows dopamine transporter (DAT) immunohistochemistry in the striatum following sham- or 60HDA-treatment. 60HDA reduced percentage of the image that was immunopositive (Panel A) and reduced mean pixel intensity of immunofluorescent pixels (Panel B). Treatment with bexarotene beginning 72 hours after 60HDA lesion significantly increased both measures. Data were analyzed with one-way ANOVAs followed by Bonferroni's post hoc comparisons. * indicates a significant difference from Sham, $p<0.05$; + indicates a significant difference from vehicle/6OHDA, $p<0.05$.

Figure 8 illustrates that compared with sham controls, 60HDA treated animals (Lesion/Veh) displayed a reduced number of TH positive cells in the SN (Panel A), reduced mean cell size (Panel B), and a reduced colocalization of TH with the neuronal marker Neurotrace and that treatment with bexarotene beginning 72 hours after 60HDA lesion (LesionBex(72)) significantly improved the number of TH positive cells, cell size and colocalization of TH and Neurotrace (Panel C).

Figure 9 shows dose effect curve of bexarotene (denoted bexarotene in the figure) and BDNF (50 ng/ml) on TH positive neurons (a), on total TH neurite length (b), and TH positive neurons displaying neurites (c), when applied after a 24h MPP+ injury (4 $\mu$M) expressed in percentage of control. (mean $\pm$ s.e.m). * : $p < 0.05$ groups vs MPP+; # MPP+ vs Control.

Figure 10 shows representative pictures of the neurotrophic effect observed in Example 6.

Figure 11 shows bexarotene effects on serum triglyceride levels. Rats were administered bexarotene either through continuous infusion of bexarotene solutions at the indicated concentrations through intracranial pumps (i.c.v., 0.1 mM, 0.3 mM and 1 mM correspond to 0.000625, 0.002, and 0.00625 mg/kg/day) for either 4 days (D4) or 8 days (D8), or as once daily oral (P.O.) doses at 1, 3, 10, 30 or 100 mg/kg/day for 5 days. At the end of the indicated dosing periods, blood was harvested, and the serum analyzed for triglyceride levels by IDEXX Corporation.

Figure 12 shows bexarotene effects on serum T4 levels. Rats were administered bexarotene either through continuous infusion of bexarotene solutions at the indicated concentrations through intracranial pumps (i.c.v., 0.1 mM, 0.3 mM and 1 mM correspond to 0.000625, 0.002, and 0.00625 mg/kg/day) for either 4 days (D4) or 8 days (D8), or as once daily oral (P.O.) doses at 1, 3, 10, 30 or 100 mg/kg/day for 5 days. At the end of the indicated dosing periods, blood was harvested, and the serum analyzed for T4 levels by IDEXX Corporation.

Figure 13 shows the interspecies correlation of AUC with bexarotene dose. AUC values derived from PK experiments using oral doses of Bexarotene. Linear regression was fitted through x=0 and y=0. The correlation coefficient is excellent ($r^2 > 0.99$). Thus one can extrapolate AUC between species. Data were taken from Targretin NDA #21055.

Figure 14 shows that AUC is proportional to bexarotene dose in humans. AUC values derived from PK experiments using bexarotene dosed orally to human subjects, once daily for 29 days A) or for 15 days B). Linear regression

was fitted through x=0 and y=0. The correlation coefficient ($r^2$) was 0.998 and 0.922 for A) and B), respectively. Thus one can predict human dose from AUC. Data taken from Miller et al., J. Clin. Oncol. 1997.

EXAMPLES

Example 1: Screening of Test Compounds in an Assay Using Nurr1 Receptor BRET assays

We have established intramolecular and intermolecular BRET (Bioluminescence Resonance Energy Transfer) assays of Nurr1 and RXR (Retinoic receptors such as, RXR-alpha, RXR-beta, and RXR-gamma) receptors by tagging each receptor with either Green Fluorescent Protein (GFP2) or Renilla luciferase (Rluc) or both tags together (see **Figure 1**). BRET occurs only when the Rluc moiety is within 100 angstroms of the GFP moiety (Pfleger and Eidne, 2003), thus these assays enable us to test each receptor for ligand-induced rearrangement of its tertiary and quartenary structures as disclosed in Figure 1. BRET assays were performed as described (Tan et al., 2007) in the following: HEK293T cells cultured in 10 $cm^2$ plates were transiently transfected with plasmid DNAs expressing a bioluminescence donor (1 $\mu$g plasmid DNA) expressing a receptor carboxy-terminally tagged with Renilla luciferase and a fluorescence acceptor (20 $\mu$g plasmid DNA) expressing a receptor amino-terminally tagged with GFP2. The receptors were Nurr1 and RXR, each was tagged with Rluc, GFP2, or both tags as indicated in Figure 1. Two days after transfection, cells were harvested and resuspended in BRET buffer (PBS containing 0.1% D-glucose and 1 mM sodium pyruvate) to a concentration of 2 x $10^6$ - 4 x $10^6$ cells/mL depending on transfection efficiency. 50 $\mu$l of 3-fold concentrated ligand dilutions were dispensed into wells of white, flat-bottomed, 96-well plates (Costar; Coming Life Sciences, Acton, MA). Ligands were incubated for 20 to 30 min with 50 $\mu$l of cell suspension to stimulate the interaction ofReceptor-Luc (bioluminescence donor) with Receptor-GFP2 (fluorescence acceptor). The BRET-2 signal was detected directly after injecting 50 $\mu$l/well of 15 $\mu$M coelenterazine 400A (DeepBlueC; PerkinElmer Life and Analytical Sciences) diluted in PBS using a Mithras LB 940 plate reader (Berthold Technologies, Bad Wildbad, Germany). After 1 s of plate-shaking, luminescence emissions for Renilla luciferase and GFP2 were recorded through BRET-optimized filters (luciferase peak 410 nm; GFP2 peak, 515 nm) for 1 to 2 s. The BRET-2 signal was calculated as the ratio between the luciferase and the GFP2 emission corrected by the background emission of non-transfected cells.

A collection of ligands with diverse chemical structures and diverse reported pharmacological profiles (see Table 1) in the BRET assays described above were profiled.

**Table 1. Compound collection.** Shown are some of the compounds profiled in these studies along with their proposed pharmacologies and primary references.

| Name | Structure | Reported Pharmacology | Reference |
|---|---|---|---|
| 9-cis-Retinoic Acid | 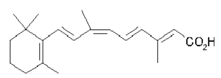 | Non-selective full retinoid agonist | |
| Bexarotene (Targretin / LGD1069) | 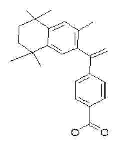 | RXR selective agonist selective | Boehm et al., J. Med. Chem., 1994. |
| LG100268 | 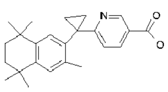 | RXR selective agonist | Boehm et al., J. Med. Chem., 1995. |
| SR11237 | 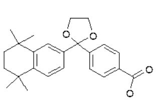 | RXR selective agonist | Wallen-Mackenzie et al., Genes Dev., 2003. |
| XCT0135908 | 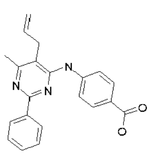 | RXR-Nurr1 heterodimer selective agonist | Wallen-Mackenzie et al., Genes Dev., 2003. |

(continued)

| Name | Structure | Reported Pharmacology | Reference |
|---|---|---|---|
| HX630 | 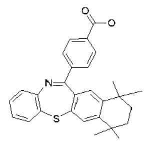 | RXR selective agonist/ potentiator | compound 29 in Umemiya et al., J. Med. Chem.,1997. |
| PAO24 | 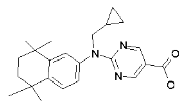 | RXR selective agonist/ potentiator | compound 10G in Ohta et al., J. Med. Chem., 2000. |
| AC-261066 | 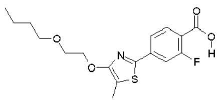 | RARb2 selective agonist | Lund et al., J. Med. Chem., 2005. |
| AC271251 | 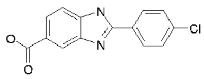 | Putative Nurr1 agonist | compound 11 in Dubois et al., Chem. Med. Chem., 2006. |
| AC271252 | 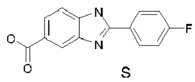 | Putative Nurr1 agonist | compound 12 in Dubois et al., Chem. Med. Chem., 2006. |
| 6-MP | 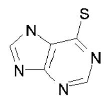 | Putative Nurr1 agonist / anticancer drug | Ordentlich et al., J. Biol. Chem., 2003. |
| 6-MP 2-deoxyribose | 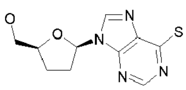 | 6-MP active metabolite | Ordentlich et al., J. Biol. Chem., 2003. |
| 6-MP ribose | 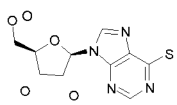 | 6-MP active metabolite | Ordentlich et al., J. Biol. Chem., 2003. |

The results, which demonstrate the agonist activity of the compounds described herein, are presented in Figure 2 and Table 2. Ligands with diverse chemical and pharmacological profiles in BRET and observed clear examples of ligands with bias for and against formation of Nurr1-RXR heterodimers as is disclosed in Figure 2 were profiled.

**Table 2. Pharmacological profiling in BRET assays.**

| | RXR-Luc & Nurr1-GFP | | RXR-Luc & RXR-GFP | | Luc-RXR-GFP | |
|---|---|---|---|---|---|---|
| Ligand | pEC50 | Eff | pEC50 | Eff | pEC50 | Eff |
| 9-cis-RA | 7.1 | 100 | 6.0 | 100 | 6.3 | 100 |
| Bexarotene | 7.9 | 105 | 6.7 | 94 | 6.9 | 93 |
| HX630 | 5.1 | 99 | 4.9 | 97 | 5.0 | 123 |
| PA024 | 7.0 | 64 | 5.9 | 87 | 6.0 | 94 |
| XCT013590 8 | 6.3 | 50 | 7.2 | 31 | 6.9 | 25 |
| AC261066 | - | 6 | 5.2 | 65 | 5.1 | 85 |

AC-271251, AC-271252, 6-MP, 6-MP 2-deoxyribose, and 6-MP ribose were inactive in all assays. Potency is reported as the negative logarithm of the EC50 (pEC50).

Surprisingly the potent RXR-selective rexinoid bexarotene (Targretin) displayed the greatest selectivity and potency in promoting formation of Nurr1-RXR heterodimers (Figure 2). The structurally related RXR agonists LG100268

and SR11237, showed similar selectivity to bexarotene in promoting formation of Nurr1-RXR heterdimers (not shown). XCT0135908, known as a selective Nurr1-RXR agonist (Wallen-Mackenzie et al, 2003), had greater maximum effect at Nurr1-RXR but was not more potent than at RXR-RXR. A structurally different rexinoid called HX630 (Umemiya et al, 1997) was equipotent at Nurr1-RXR and RXR-RXR, while the RARb2-selective compound AC-261066 (Lund et al, 2005) was active only at RXR-RXR. Surprisingly the putative Nurr1 agonists compounds 11 & 12 (Dubois et al, 2006) and 6-MP, 6-MP-ribose, and 6-MP-2-deoxyribose (Ordentlich et al, 2003) were inactive at all receptor combinations tested (data not shown).

We have enabled assays to detect ligand-induced gene transcription (reporter gene assays) in order to confirm results obtained in BRET2 assays. Gene transcription is quantified by luciferase expression which is driven by response elements that respond to different nuclear receptors: the Retinoid X Receptor (RXR) response element RXRE, the Retinoic Acid Receptor (RAR) response element RARE, and the NGFI-B response element, NBRE, which is bound by Nurr1 monomers (Castro et al., 1999). We tested bexarotene in these assays and observed that its activity at RXRE and NBRE response elements, but not RARE response elements was increased substantially relative to the non-selective retinoid 9-cis retinoic acid when Nurr1 was co-expressed (**Table 3** and **Figure 3**) A similar pattern was seen with PA024, HX630 and XTC0135908.

**Table 3. Pharmacological profiling in mammalian reporter gene transcription assays.** Potency is given in units of nanomolar. Maximum response is given as Eff% and is normalized to the response of 9-cis retinoic acid.

| | pNBRE | | pNBRE & Nurr1 | | pRXRE | | pRXRE & Nurr1 | | pRARE | | pRARE & Nurr1 | |
|---|---|---|---|---|---|---|---|---|---|---|---|---|
| Ligand | $EC_{50}$ (nM) | Eff% | $EC_{50}$ (nM) | Eff% | $EC_{50}$ (nM) | Eff% | $EC_{50}$ (nM) | Eff% | $EC_{50}$ (nM) | Eff% | $EC_{50}$ (nM) | Eff% |
| 9-cis-RA | 564 | 100 | 106 | 100 | 88 | 100 | 105 | 100 | 154 | 100 | 208 | 100 |
| Bexarotene | 20 | 28 | 17 | 83 | 144 | 44 | 41 | 65 | 30 | 19 | 26 | 23 |
| PA024 | 43 | 24 | 45 | 83 | 131 | 44 | 106 | 64 | 101 | 19 | 53 | 20 |
| HX630 | - | 16 | 18 | 41 | 664 | 26 | 2460 | 47 | - | 8 | - | 6 |
| XTC0135908 | - | 10 | 25 | 25 | - | 4 | 5321 | 49 | - | 3 | - | 0 |

Example 2: Bexarotene protects neurons

Based on the selective Nurr1-RXR profile of bexarotene, bexarotene was tested for the ability to protect against 6-OHDA (6-hydroxydopamine) induced neuronal loss in rodents. The results are shown in Figure 4. Male Sprague-Dawley rats were implanted with bilateral guide cannulas 2 mm above the SN. 5-7 days post-surgery, subjects received treatments which consisted of 3 daily microinjections of bexarotene (1μL of 10 μM) or vehicle. 4 hrs following the second bexarotene treatment, subjects were injected with vehicle or 6-OHDA (4 μL of 2 mg/ml) to induce loss of DA neurons. 48 hrs after the final microinjection, subjects were sacrificed. Their brains were fixed, sectioned through the SN and labeled for tyrosine hydroxylase. Bilateral serial sections (3/side, -5.2 mm from bregma) were photographed and analyzed for the number of TH+ neurons and the % of the section that was immunopositive. 6-OHDA treatment (Lesion) produced a decrease in DA cell number and % of the section that was immunopositive relative to vehicle treated controls (Sham). As shown in Figure 4, microinjected bexarotene completely prevented the loss of dopaminergic cells induced by 6-OHDA.

It could be concluded that there is a strong correlation between formation of Nurr1-RXR heterodimers in BRET and neuroprotection of DA (dopaminergic) neurons in models ofPD (Parkinson's disease), with bexarotene being very effective in both.

Example 3: Bexarotene concentrations in plasma and brain administered peripherally or centrally

Bexarotene was administered peripherally by once daily oral dosing (P.O. (QD), peripherally by continuous infusion sub-cutaneously (s.c.) (C.I. s.c.) using implanted pumps, and centrally by continuous infusion intracerebroventricularly (i.c.v.) using pumps implanted intra-cranially. The brain to plasma ratio was much higher with i.c.v. administration, reaching a ratio of 6 at 0.00625 mg/kg/day and estimated to be greater than 9 at 0.002 mg/kg/day. The brain levels were 12 ng/g at 0.00625 mg/kg/day compared with 2 ng/ml (equal to 2 ng/g) in plasma. Significantly, 0.00625 mg/kg/day administered C.I. i.c.v. was effective in reversing the neuronal and behavioral deficits following 6-hydroxydopamine (60HDA) lesions of the substantia nigra pars compacta (SNc) (see below). Similarly, a dose of 0.25 mg/kg/day administered C.I. s.c. resulted in brain bexarotene levels of 14 ng/g, suggesting that 0.25 mg/kg/day administered C.I. s.c. would also be an effective dose in reversing the neuronal and behavioral deficits following 6-hydroxydopamine (60HDA) lesions of the substantia nigra pars compacta (SNc). However in this case the plasma levels of bexarotene were 12 ng/g; resulting in a brain/plasma ratio of 1.2. Finally a series of doses of bexarotene ranging from 1 to 100

mg/kg/day were administered as once daily oral doses (P.O. QD). Brain and plasma levels of bexarotene increased with dose in a dose-proportional manner from 1 to 10 mg/kg/day and in a slightly less than dose-proportional manner at 30 and 100 mg/kg/day. The brain/plasma ratio was consistently below 1, ranging between 0.4 and 0.8 at all doses tested.

The results are shown in Table 4.

**Table 4**

| Route | solution conc | mg/kg/day | Expt | time | plasma (ng/ml | brain (ng/g) | Brain/plasma |
|---|---|---|---|---|---|---|---|
| **C.I. i.c.v.** | 0.1 mM | 0.000625 | | 4 days | BQL | | |
| | | | | 8 days | BQL | BQL | |
| | 0.3 mM | 0.002 | | 4 days | BQL | | |
| | | | | 8 days | BQL | 9 +/-1 | >9* |
| | 1 mM | 0.00625 | | 4 days | BQL | | |
| | | | | 8 days | 2- | 12 +/-2 | 6.0 |
| **C.I.s.c.** | 16 mM | 1 | #1 | 4 days | 30 +/-21 | | |
| | | | | 8 days | 41 +/-2 | 47 +/-5 | 1.1 |
| | | | #2 | 4 days | 28 +/-7 | | |
| | | | | 8 days | 39 +/-9 | 48 +/-8 | 1.2 |
| | 4 mM | 0.25 | #2 | 4 days | 12 +/-11 | | |
| | | | | 8 days | 12 +/-6 | 14 +/-6 | 1.2 |
| **P.O. (QD)** | | 1 | | 5 days, 3 hr | 80 +/-18 | 38 +/-6 | 0.5 |
| | | 3 | | 5 days, 3 hr | 254 +/-147 | 100 +/-44 | 0.4 |
| | | 10 | | 5 days, 3 hr | 725 +/-214 | 369 +/-195 | 0.5 |
| | | 30 | | 5 days, 3 hr | 1138 +/-280 | 855 +/-396 | 0.8 |
| | | 100 | | 5 days, 3 hr | 1500 +/-208 | 1263 +/-268 | 0.8 |

*based on a detection limit of 1 ng/ml in plasma. C.I. i.c.v. and C.I. s.c. doses in mg/kg/day based on 6 ul/day (i.c.v.) or 60 ul/day (s.c.) solutions delivered at the indicated solution concentrations. C.I.i.c.v. means continuous infusion, intracerebroventricularly for 4 or 8 days as indicated.
C.I.s.c. means continuous infusion, sub-cutaneously, for 4 or 8 days as indicated.
P.O. (QD) is once daily oral administration. 5 days, 3 hr means once daily administration for 5 days with samples taken 3 hours after the final dose.

Example 4: Bexarotene efficacy when administered i.c.v.

This example illustrates evaluation ofbexarotene efficacy when administered i.c.v. after 6-OHDA leasion to assess neuroregenerative potential ofbexarotene. The endpoints assessed were:

- Neuroprotection measured by tyrosine hydroxylase (TH) staining in the substantia nigra (SNc) and dopamine transporter (DAT) staining in the striatum (STR).
- Behavioral assessments including rotarod, challenging beam, and spontaneous locomotion

Bexarotene was tested for its ability to reverse neuronal and behavioral deficits following 6-hydroxydopamine (60HDA) lesions of the substantia nigra pars compacta (SNc). 60HDA was infused bilaterally into the SNc of male rats to produce destruction of dopamine neurons. Using an osmotic pump, bexarotene or vehicle was infused into the cerebral ventricle at a constant rate (0.25 μL/hr or 6 μL/day of a 1 mM solution of bexarotene providing a dose of 0.000625 mg/kg/day, see Table 4 above) for 28 days beginning 72 hours after 60HDA infusion. Following the 28 days of treatment, animals were assessed in 3 tests of coordinated motor function (spontaneous locomotion, rotorod and challenging beam) and then tissue was collected to assess tyrosine hydroxylase immunofluorescence in the substantia nigra. Treatment with bexarotene reversed behavioral deficits caused by 60HDA administration (see Figure 5), and resulted in improved tyrosine hydroxylase expression in the SNc (see Figure 6) and improved dopamine transporter expression in the STR (see Figure 7).

This example indicates that bexarotene displays efficacy in both neuroregeneration and behavioral endpoints when administered after 6-OHDA lesioning.

**Methods**

*Subjects:* The subjects for these experiments were male Sprague-Dawley rats purchased from Charles Rivers Laboratories (Hollister, CA) weighing 225-250 g upon arrival. Rats were housed in pairs in polypropylene cages within a temperature controlled vivarium maintained on a 12 hr light:dark cycle (lights on 7 am). For the duration of the experiments, animals received free access to food and water. All procedures were conducted in accordance with the NIH *Guidelines for the Care and Use of Laboratory Animals* and were approved by the Institutional Animal Care and Use Committee (IACUC) at ACADIA Pharmaceuticals. Animals were acclimated to vivarium conditions and handling for a minimum of one week prior to surgery.

*Surgery:* In order to protect norepinephrine terminals, each animal received an injection of desipramine (10 mg/kg) about 15 min prior to being anesthetized using isofluorane. Animals were placed into a stereotaxic apparatus and bilateral infusions of 60HDA (8μg/4μl) or 0.2% ascorbic acid vehicle were aimed at the SNc (A/P -5.0 mm, M/L ±1.6 mm, D/V -8.2 mm relative to bregma). After 60HDA infusions, an Alzet osmotic pump (Durect Corporation, Cupertino, CA) attached to an intracranial guide cannula was implanted subcutaneously between the shoulder blades of each animal. The guide was placed intracerbroventricularly (i.c.v., A/P -0.8 mm, M/L -1.4 mm, D/V -4.5 mm relative to bregma) and was attached to the skull with jeweler's screws and dental acrylic and the incision was closed with staples. Animals received supportive care following surgery, including administration of subcutaneous (sc) fluids (10 ml/day) and soft food mashes, until they surpassed their surgical weights. Subjects were allowed at least 28 days prior to behavioral testing:

*Pumps:* The osmotic pumps (Alzet, model 2004) were weighed and then filled with bexarotene (1 mM) or vehicle (1% DMSO in saline) 48 hours prior to surgery. They were then incubated in 0.9% physiological saline at 37C until surgically implanted. The pumps infused at a rate of 25 μL/hr for 28 days after implantation. Infusion pumps were connected to the i.c.v. cannula with vinyl tubing and different infusion conditions were achieved by filling the tubing with varying amounts of vehicle before bexarotene reached the guide. Thus, bexarotene infusion began 72 hours after implantation of the guide and the following surgery/treatment conditions were employed (N=3-5/group): Sham/vehicle, Sham/bexarotene(72), 60HDA/vehicle, 60HDA/bexarotene(72). After completion of the experiment, a subset of the osmotic pumps was removed. The pumps were weighed and aspirated in order to verify compound delivery. For all pumps tested, this procedure confirmed that the pumps successfully delivered compound.

*Spontaneous Locomotion:* Locomotor activity studies were conducted in acrylic chambers (42 cm x 42 cm x 30 cm) equipped with 16 infrared photobeams along each horizontal axis (front-to-back and side-to-side) from Accuscan Instruments, Inc. (Columbus, OH). Animals were placed into the chamber for 15 min and their activity (distance traveled) was recorded.

*Rotorod:* Rotorod testing was conducted on a rotating cylinder (70 mm diameter) with knurled tread to aid in gripping. Animals were placed on the cylinder and it was set to rotate at 1 rpm for 15 sec. If animals fell or jumped from the cylinder within 30 sec., they were replaced and the acclimation period restarted. Once animals successfully remained on the cylinder for the acclimation period, the speed of rotation was increased 1 rpm every 15 sec to a maximum of 10 rpm. The time in seconds that animals remained on the cylinder after the acclimation period and the maximum rpm achieved were recorded. A second trial was conducted after a 2 min intertrial interval using the same procedure, but the acclimation period was decreased such that animals were only required to step with all four feet before the speed of rotation was increased.

*Challenging Beam Test:* The challenging beam test was conducted on a 102 cm long bi-level beam made from ABS plastic. The top, narrower beam gradually tapered from 3.5 cm to 0.7 cm, while the bottom, wider beam gradually tapered from 5 cm to 1.8 cm along the length of the beam. The beam was elevated 23 cm above the table. Animals received five training trials. On the first training trial animals were placed at the end of the beam and were required to jump into a holding tub. On successive trials, animals were placed 25, 50, 75 and 100 cm from the end of the beam and were required to traverse the beam and jump into the holding tub at the end. Following training, a single test trial was conducted where each animal was placed at the beginning of the beam and the start latency (time required to move all four feet from their starting locations) and run time (time required to traverse the beam after starting) were recorded. Animals were allowed a maximum of 300 seconds to traverse the beam, at which point they were removed from the beam and a run time of 300 sec was recorded.

*Tyrosine Hydroxylase Fluorescent Immunohistochemisty:* Following behavioral testing, animals were anesthetized and perfused transcardially with PBS followed by 4% paraformaldehyde. Fixed tissue brains were sectioned (50 μm) through the subtantia nigra and then were immunolabeled for tyrosine hydroxylase using the following steps: 3 X 5min rinses in 1X phosphate buffered saline (PBS); 45 min blocking step in blocking buffer (0.8 PBS, 3% normal donkey serum, 0.1% Triton); incubation with rabbit anti-tyrosine hydroxylase polyclonal antibody (AB 152, Millipore Corp., Billerica, MA) in working buffer (1x PBS, 1% blocking buffer, 0.1% Triton) for 2hr at room temperature; 3 X 5min rinses in working buffer; incubation with donkey anti-rabbit Alexa Fluor 488 fluorescent secondary antibody (A21206, Invitrogen Corp., Carlsbad, CA) in working buffer for 1hr; 3 X 5min rinses in working buffer.

*Dopamine Transporter Immunohistochemistry:* Similarly, fixed brains were sectioned (50 μm) through the striatum and labeled for the dopamine transporter. The dopamine transporter was labeled with DAB immunohistochemistry using the following steps: 3 X 5min rinses in 1X phosphate buffered saline (PBS); 20 min incubation in sodium citrate buffer (10mM sodium citrate in 1 X PBS, 0.05% Tween 20, pH=6.0) at 80C to promote antigen retrieval; 10 min incubation in 3% hydrogen peroxide to block peroxidase binding sites; 1 hour protein blocking step in blocking buffer (1 x PBS, 8% normal goat serum, 3% bovine serum albumin, 0.1% Triton, avidin blocking solution from Vector Laboratories, Burlingame, CA); incubation with rat anti-dopamine transporter monoclonal antibody (MAB369, Millipore Corp.) in a working buffer (1x PBS, 2% normal goat serum, 1% bovine serum albumin, biotin blocking solution from Vector Laboratories) overnight at 4C; 3 X 5min rinses in 1 X PBS; incubation with goat anti-rabbit biotinylated secondary antibody (BA-9400, Vector Laboratories) in working buffer without biotin for 1hr; 3 X 5min rinses in 1 X PBS; 30 min incubation in ABC wash (PK-6100, Vectastain Elite ABC kit, Vector Laboratories); 3 X 5min rinses in 1 X PBS; 5 min DAB incubation (SK-4100, DAB substrate kit, Vector Laboratories); 1X5 5 min rinse in $ddH_2O$; 2 X 5 min rinses in 1 X PBS. The sections were then mounted on slides and allowed to dry before being submerged in successive 3 min washes (70%EtOH, 95% EtOH, 100% EtOH, 50/50 Citrisolve/EtOH, 100% Citrisolve) and coverslipped using a xylene-based permanent mounting medium (H-5000, VectaMount, Vector Laboratories).

After immunolabeling sections were mounted and coverslipped using fluorescent antifade mounting medium (S3023, Dako USA, Carpinteria, CA). Single optical plane images were obtained using an Olympus BX51 Fluorescent microscope (Olympus America Inc., Center Valley, PA) equipped with a digital camera (Retina 2000R, Qimaging, Surrey, BC). Images were acquired using a 4X air objective (UPlanFLN, N.A. 0.13) with 2X digital magnification. For each animal 3 consecutive sections through each SN were analyzed (-5.2 mm relative to bregma according to the atlas of Paxinos and Watson, 1997). All images (N=6/animal) were treated as independent observations and analyzed by an observer blind to each subject's treatment condition using *ImageJ* software (available at http://rsb.info.nih.gov/nih-imageJ, developed by Wayne Rasband at NIH, Bethesda, MD) in order to determine the cell count, cell size (pixels/cell), pixel intensity, and % immunopositive. Data represent the mean SN section for these measures across different treatment conditions. Controls were performed via omission of the primary antibody and revealed no non-specific staining (data not shown).

*Confirmation of drug delivery.* Animals receiving bexarotene i.c.v. by osmotic pumps were sacrificed at 3 weeks, brains harvested, and analyzed for bexarotene using LC-MS/MS according to the vendor's (Agilux Laboratories) procedures. The results of this study are shown in Table 4 above.

<u>Example 5: Bexarotene regenerates neurons</u>

In this example (see Figure 8) animals received vehicle (Sham-All Tx) or 60HDA treatment (Lesion) bilaterally into the SN. 3 days after surgery, animals were either sacrificed (Day 3) or began receiving bexarotene (1mM, 0.25 μL/hr) or vehicle (1% DMSO) intracerebroventricularly for 28 days. Compared with sham controls, 60HDA treated animals (Lesion/Veh) displayed a reduced number of TH positive cells in the SN (Panel A), reduced mean cell size (Panel B), and a reduced colocalization of TH with the neuronal marker Neurotrace. Treatment with bexarotene beginning 72 hours after 60HDA lesion (LesionBex(72)) significantly improved the number of TH positive cells, cell size and colocalization of TH and Neurotrace. Notably, bexarotene treatment also significantly improved these measures when compared with animals sacrificed 3 days after lesion (Panel C). Data were analyzed with one-way ANOVAs followed by post hoc Tukey's multiple comparisons test. * indicates a significant difference from Sham, $p<0.05$; + indicates a significant difference from vehicle/6OHDA, $p<0.05$; ^ indicates a significant difference from Day 3, $p<0.05$.

The results are shown in Figure 8.

<u>Example 6: Effect of bexarotene after a MPP+ injury in rat primary dopaminergic neurons</u>

Parkinson's disease (PD) is the second most common neurodegenerative disorder in the United States. The predominant motor symptoms ofPD including slow movement, resting tremor, rigidity, and gait disturbance, are caused by the loss of dopaminergic neurons in the substantia nigra (SN). Although the aetiology of PD remains unknown, both genetic and environmental factors appear to play a role (Paisan-Ruiz et al., 2004; Vila & Przedborski, 2004). The neurotoxicant 1-methyl-4-phenyl-1,2,3,6-tetrahydropyridine (MPTP) is a specific dopaminergic neuronal toxin. MPTP is converted to 1-methyl-4-phenyl pyridinium (MPP+) by astroglia and then causes specific dopaminergic neuronal death in the SN, thus leading to the clinical symptoms ofPD in humans, primates and mice (Uhl et al., 1985). For this reason, MPTP-induced dopaminergic neurotoxicity in mice is widely used as a model for PD research. It has been largely reported that MPP+ causes neurodegeneration of dopaminergic neurones in vitro and provides a useful model of Parkinson's disease in vitro.

The neurotrophins brain derived neurotrophic factor (BDNF) and glial derived neurotrophic factor (GDNF) have been suggested to reduce the MPP+- induced neurodegeneration in vitro (Hung & Lee, 1996); (Hou et al., 1996).

This example investigated the restorative effect of bexarotene tested at 7 concentrations on rat primary mesencephalic cultures previously injured by a 24h exposure to 1-methyl-4-phenylpyridinium (MPP+), a Parkinson' disease model in vitro. BDNF was used as a positive control in this study.

*EXPERIMENTAL PROTOCOL*

*Primary cultures of dopaminergic neurons*

Rat dopaminergic neurons were cultured as described by Schinelli et al., 1988. Briefly pregnant female rats of 15 days gestation were killed by cervical dislocation (Rats Wistar; Janvier) and the foetuses removed from the uterus. The embryonic midbrains were removed and placed in ice-cold medium of Leibovitz (L15; PAN) containing 2% of Penicillin-Streptomycin (PS; Invitrogen) and 1% of bovine serum albumin (BSA; PAN). Only the ventral portions of the mesencephalic flexure were used for the cell preparations as this is the region of the developing brain rich in dopaminergic neurons. The midbrains were dissociated by trypsinisation for 20 min at 37°C (Trypsin EDTA 1X; PAN) diluted in PBS without calcium and magnesium. The reaction was stopped by the addition of Dulbecco's modified Eagle's medium (DMEM; PAN) containing DNAase I grade II (0.5 mg/ml; PAN) and 10% of foetal calf serum (FCS; Gibco). Cells were then mechanically dissociated by 3 passages through a 10 ml pipette. Cells were then centrifuged at 180 x g for 10 min at +4°C on a layer of BSA (3.5%) in L15 medium. The supernatant was discarded and the cells of pellet were re-suspended in a defined culture medium consisting of Neurobasal (Gibco) supplemented with B27 (2%; Gibco), L-glutamine (0.2 mM; Invitrogen) 2% of PS solution and 10 ng/ml BDNF (PAN) and 1 ng/ml GDNF.(PAN) Viable cells were counted in a Neubauer cytometer using the trypan blue exclusion test. The cells were seeded at a density of 40000 cells/well in 96 well-plates (wells were pre-coated with poly-L-lysine (greiner) and were cultured at 37°C in a humidified air (95%)/CO2 (5%) atmosphere. Half of the medium was changed every 2 days with fresh medium.

*MPP+ exposure and drug treatment: restorative protocol*

Briefly, on day 6 of culture, the medium was removed and fresh medium was added, without or with MPP+ at 4 μM. On day 7, the culture was washed with fresh medium without (containing vehicle) or with bexarotene (0.3, 1, 3, 10, 30, 100 and 300 nM) or BDNF (50 ng/ml) for 48h. After 48h with or without bexarotene (0.3, 1, 3, 10, 30, 100 and 300 nM) or BDNF (50 ng/ml), cells were fixed (all conditions) by paraformaldehyde 4 % solution. In all wells, the final concentrations were in 0.1 %DMSO. After permeabilization with 0.1% saponin (Sigma), cells were incubated with mouse monoclonal primary against tyrosine hydroxylase antibody (TH, Sigma) to stain specifically dopaminergic neurons. This antibody was revealed with Alexa Fluor 488 goat anti-mouse IgG (Molecular probe). The number of TH neurons and total neurite of TH neurons were measured in each well.

*Analysis and method of quantification*

For each condition, 2x10 pictures per well were taken in the same condition using InCell AnalyzerTM 1000 (GE Healthcare) with 10x magnification. The analyses were automatically done using developer software (GE Healthcare) to measure the total number of TH positive neurons. Two means of 10 pictures were automatically performed by well. Data were expressed in percentage of control condition. Statistical analyses (using Graph Pad Prism's package) were done on the different conditions using ANOVA test following by Dunnett's test (when allowed), significance was set for $p \leq 0.05$. Representative pictures are shown in Fig. 10.

In addition, the control/vehicle, MPP+/vehicle, MPP+/bexarotene (300 nM), and MPP+/BNDF (50 ng/ml) conditions were analyzed for number of neuron displaying neurites. For each condition, 10 pictures per well were taken in the same condition using InCell AnalyzerTM 1000 (GE Healthcare) with 10x magnification. The analyses were done manually to measure the number of TH positive neurons displaying neurites. 6 wells per conditions were analysed. Data were expressed in percentage of control condition. Statistical analyses (using Graph Pad Prism's package) were done on the different conditions using one-way ANOVA test following by Dunnett's test (when allowed: $p < 0.01$), significance was set for $p \leq 0.05$.

*RESULTS*

MPP+ at 4 μM (24h intoxication) showed a large and significant TH positive neuron and TH positive neurite decreases. bexarotene applied after the 24h intoxication displayed an increase of the total number of TH neurons at all tested concentrations. This restorative effect was significant from 10 up to 300 nM. Similarly, BDNF (50 ng/ml) was able to reverse the MPP+ injuries. It could be mentioned that for the 3 highest test concentrations (30, 100 and 300 nM), bexarotene displayed a similar effect (in survival) as the one observed with BDNF used here as reference test compound.

Similarly, a significant effect was observed on the length of TH neurite at the 2 highest concentrations of bexarotene (100 and 300 nM). The neurotrophic effect observed at the dose of 300 nM was as high as the one of BDNF (internal reference test compound). The results are illustrated in Fig. 9A, Fig. 9B, and 9C. Representative images showing the regenerative effect of bexarotene on neurons are shown in Figure 10.

Example 7: Bexarotene doses that cause side effects

Elevation of serum triglycerides and hypothyroidism are two prominent side-effects known to be caused by bexarotene. Rats were administered bexarotene over a period of several days, at a range of doses, either with continuous infusion through the i.c.v. route, or orally. As shown in Figure 11, the triglyceride levels in rats given bexarotene P.O. were increased in a dose dependent manner and were markedly elevated compared to all of the doses provided by the i.c.v. route; including the highest i.c.v. dose which was shown to effectively reverse neuronal and behavioral deficits following 6-hydroxydopamine (60HDA) lesions of the substantia nigra pars compacta (SNc) (Example 4) and regenerate dopamine neurons (Example 5). Similarly, T4 levels were significantly lower in the 100 mg/kg/day P.O. dose group compared to the lower dose groups and the i.c.v. dose groups (Figure 12). 60 mg/kg/day is considered the effective anti-cancer dose in rats (Targretin NDA #21055). Finally, at higher doses of bexarotene a decrease in body weight gain was noted (see Table 5).

**Table 5**. Bexarotene was administered once per day orally, at the indicated doses (mg/kg/day). Body weight was measured at the start and end of the dosing period and expressed as percent body weight gain.

| PO dose | %BW gain |
|---|---|
| Bex (1) | 20.8 +/-2.8 |
| Bex (3) | 19.8 +/-3.4 |
| Bex (10) | 20.4 +/-1.6 |
| Bex (30) | 11.2+/-0.8 |
| Bex (100) | 7.0 +/-3.6 |
| Veh | 20.9 +/-2.6 |

These data suggest it is possible to identify doses of bexarotene that are effective for reversing neurodegeneration that have greatly reduced side effects compared to how bexarotene is currently used clinically.

Example 8: Bexarotene doses in humans

Extrapolation of AUC and dose between species. Information provided in Targretin NDA #21055 about the pharmacokinetics ofbexarotene indicates that it is possible to extrapolate drug exposure (quantified as 'area under the curve' or AUC) between species **(Figure 13).** Furthermore, published clinical data show that there is a strong correlation between doses of bexarotene given to humans, and AUC **(Figure 14).**

Doses of bexarotene to effectively treat cancer in humans or rats. The recommended starting clinical dose of bexarotene for cancer treatment in humans is 300 mg/m$^2$/day (equivalent to 8.1 mg/kg/day or ~650 mg/day for an 80 kg person), and this dose may be increased if there is insufficient response (Targretin NDA #21055; Duvic et al., J. Clin. Oncol., 2001). The effective anti-cancer dose in rats is 60 mg/kg/day (Targretin NDA #21055).

Effective doses ofbexarotene in a rat model of Parkinson's disease are much lower. We have shown bexarotene administered with continuous infusion through the intracerebroventricular route (C.I. i.c.v.) has regenerates neurons in rats previously given the neurotoxin 60HDA (see **Figure 5-8).** The brain concentration at this dose ofbexarotene was 12 ng/g (see **Table 4** above). Delivery of 0.25 mg/kg/day of bexarotene systemically using continuous infusion through the sub-cutaneous route (C.I. s.c.) provides a very similar bexarotene brain concentration of 14 ng/g **(Table 4).** Thus assuming bexarotene delivered at 0.25 mg/kg/day C.I. s.c. would also regenerate dopamine neurons damaged by 60HDA, one can use the plasma levels of bexarotene delivered at 0.25 mg/kg/day C.I. s.c., which were 12 ng/ml, to calculate AUC in rats for an effective PD dose delivered systemically.

Thus using the ratio of AUC in rats for an effective cancer dose to an effective PD dose, one may extrapolate the AUC observed in humans at effective cancer doses to the AUC needed for efficacy in PD. One can then estimate doses of bexarotene needed for efficacy against PD in humans using the human AUC/dose correlation(s) above.

Estimate bexarotene dose reduction to treat PD instead of cancer in humans

Human data

- Recommended anti-cancer dose: 300 mg/$m^2$ = 8.1 mg/kg or 648 mg/day[1]
- [2]AUC at recommended anti-cancer dose: 7.6 μM*hr
- [3]AUC at recommended anti-cancer dose: 10.9 μM*hr
- [4]AUC at recommended anti-cancer dose: 15.9 μM*hr

Rat data

- [3]Effective anti-cancer dose: 60 mg/kg/day
- [3]AUC at 30 mg/kg/day: 23 μM*hr
- AUC at 60 mg/kg/day (interpolated): 34 μM*hr
- [3]AUC at 100 mg/kg/day: 45 μM*hr
- [5]Brain level at effective PD dose: 12 ng/g
- Dose producing 14 ng/g brain levels: 0.25 mg/kg/day (C.I. s.c., Table 4)
- AUC at effective PD dose[6]: 0.8 μM*hr

[1]based on an 80 kg person; [2]AUC value from Miller et al, J. Clin. Oncol. 1997; [3]AUC values from Targretin NDA #21055; [4]AUC value from Duvic et al., J. Clin. Oncol. 2001; [5]*Brain* level produced by continuous infusion i.c.v. of 0.00625 mg/kg/day or by continuous infusion s.c. of 0.25 mg/kg/day (see Table 4). Note that continuous infusion i.c.v. of 0.00625 mg/kg/day was effective in reversing the behavioral and neural effects of 60HDA (see Fig 5-8). [6]AUC calculated using the trapezoidal method based on plasma levels of 12 ng/ml at 0.25 mg/kg s.c. (see Table 4).

Effective doses of bexarotene to treat Parkinson's disease in humans may be estimated as follows:

$$(\text{Rat AUC}_{\text{Parkinson's}} \div \text{Rat AUC}_{\text{cancer}}) \times \text{Human AUC}_{\text{cancer}} = \text{Human AUC}_{\text{Parkinson's}}$$

Using the correlation of human AUC to human dose in Figure 14A or 14B:

$$\text{Human AUC}_{\text{Parkinson's}} \div \text{slope} = \text{Human Dose}_{\text{Parkinson's}}$$

A summary of these calculations is provided in Table 6.

## Table 6

| | Cancer (rat) | | Parkinson's (rat) | | Cancer (human) | | Parkinson's (human) | | |
|---|---|---|---|---|---|---|---|---|---|
| Treatment: | dose | AUC | dose | AUC | AUC | **dose** | AUC | **dose** | |
| Units: | mg/kg | (μM * hr) | mg/kg | (μM * hr) | (μM * hr) | mg/day | (μM * hr) | mg/day | |
| Column: | A | B | C | D | E | G | F | H | I |
| Operation: | - | - | - | - | - | - | (D × E ÷ B) | (E ÷ m × 80) | (E ÷ m × 80) |
| | 30 | 23 | 0.25 | 0.8 | 10.9 | **648** | 0.38 | **23** | **34** |
| | 60 | 34 | 0.25 | 0.8 | 10.9 | **648** | 0.26 | **16** | **23** |
| | 100 | 45 | 0.25 | 0.8 | 10.9 | **648** | 0.19 | **12** | **17** |

A: Rat effective dose (cancer) of 60 mg/kg = effective anti-cancer dose in rats (from Targretin NDA #21055).
B: Rat effective dose (PD) of 0.25 mg/kg administered as s.c. continuous infusion provides brain Bexarotene exposure (Table 4) sufficient to neuroregenerate neurons in rats given 6OHDA (see Figs 5-8).
C: Rat AUC (cancer) at 30 and 100 mg/kg P.O. from Targretin NDA #21055 and confirmed experimentally. Rat AUC (cancer) at 60 mg/kg interpolated from AUCs at 30 and 100 mg/kg.
D: Rat AUC (PD) calculated from plasma levels in rats given 0.25 mg/kg s.c. (Table 4) using the trapezoidal rule
E: Human AUC (cancer) at 300 mg/$m^2$ P.O. (equivalent to 8.1 mg/kg) from Targretin NDA #21055.
F: Human AUC (PD) calculated as (human $AUC_{cancer}$ x Rat $AUC_{PD}$ ÷ Rat $AUC_{cancer}$)
G: Human starting dose (cancer) based on 300 mg/$m^2$ dose (8.1 mg/kg/day x 80 kg person).
H: Human dose (PD) estimated using human AUC (PD) divided by m (slope of Figure 14A) x 80 kg
I: Human dose (PD) estimated using human AUC (PD) divided by m (slope of Figure 14B) x 80 kg

The AUC at the recommended cancer dose of 300 mg/m2 (equal to 8.1 mg/kg/day or 648 mg/day for an 80 kg person) has been reported to be as high as 15.9 (Duvic et al., J Clin Oncol, 2001) and as low as 7.6 (Miller et al., J Clin Oncol, 1997). Using these values, instead of 10.9 as in **Table 6** above, the estimated effective Parkinson's doses of bexarotene in humans are:

**Table 7:** Dose estimates based on AUC values at the recommended human cancer dose.

| AUC basis: | 10.9 μM*hr | 15.9 μM*hr | 7.6 μM*hr |
|---|---|---|---|
| High estimates: | 23 to 34 | 34 to 50 | 16 to 24 |
| Mid estimates: | 16 to 23 | 23 to 34 | 11 to 16 |
| Low estimates: | 12 to 17 | 17 to 26 | 8 to 12 |

Dose estimates are given in mg/day. Ranges based on estimates using two different slopes relating human AUC to dose (see Figure 14A and 14B and Table 6, column H and I).

The recommended dose for Targretin in humans to treat cancer is 300 mg/$m^2$, equivalent to ~650 mg/day for an 80 kg person. However optimum therapy in many patients requires even higher doses such as 400 mg/m2, equivalent to ~860 mg/day for an 80 kg person (Duvic et al, 2001; Gniadecki et al, 2007). Based on the analysis provided above, doses that would be efficacious in humans afflicted with neurodegenerative disease are predicted to be:

- **8 - 50 mg/day (the lowest and highest estimates in Table 7 above), a 13 to 78-fold reduction in dose** compared to a dose of 650 mg/day to treat cancer in humans, the recommended starting dose.
- **11 - 67 mg/day** using a basis of a dose of 860 mg/day rather than 650 mg/day to treat cancer in humans, a more optimum dose in many subjects.

The smallest available Targretin pill is 75 mg, 9-fold lower than the starting dose to treat cancer in humans.

**Intracerebroventricular (i.c.v.) administration of Bexarotene offers additional advantages**

At least two additional benefits may be realized with i.c.v. administration of Bexarotene:

- Very low doses will be effective
- Brain:plasma ratio of drug will increase further reducing systemic drug exposure

**Table 4** (shown above) reveals that the brain/plasma ratio is much higher with i.c.v. administration, reaching effective brain concentrations while keeping peripheral levels low.

The brain concentration achieved with 0.25 mg/kg/day of s.c. administration (12 ng/g); was also achieved with 0.00625 mg/kg/day of i.c.v. administration, **a 40-fold lower dose.** Significantly, this level of brain exposure had neuroregenerative effects in a rat model ofPD (see **Figure 5-8).** Using a plasma level of 2 ng/ml **(Table 4),** the AUC with i.c.v. administration was at least **6-fold lower** than with s.c. administration while providing equal brain exposure. Using the ranges provided in Table 7, compared to the recommended dose for Targretin in humans to treat cancer (300 mg/$m^2$, equivalent to ~650 mg/day for an 80 kg person) the effective dose ofbexarotene administered i.c.v. to humans are estimated to be:

- AUC basis (6x): effective i.c.v. dose of bexarotene is 1.3 to 8.3 mg/day
- Dose basis (40x): effective i.c.v. dose of bexarotene is 0.2 to 1.3 mg/day

Compared to the optimum dose for Targretin in humans to treat cancer in some patients (400 mg/$m^2$, equivalent to ~860 mg/day for an 80 kg person) the effective doses of bexarotene administered i.c.v. to humans are estimated to be:

- AUC basis (6x): effective i.c.v. dose of bexarotene is 2 to 11 mg/day
- Dose basis (40x): effective i.c.v. dose of bexarotene is 0.3 to 1.7 mg/day

Use of FDA Guidance for Industry: Estimating the Maximum Safe Starting Dose in initial Clinical Trials for Therapeutics in Adult Healthy Volunteers

Another means to estimate effective doses to treat PD in humans is to use FDA recommended methods of extrapolating between human and animal dosing data. This FDA publication can be found at:

www.fda.gov/downloads/Drugs/GuidanceComplianceRegulatoryInformation/Guidan ces/ucm078932.pdf

Using the conversion factor for rat doses to human equivalent dose provided in Table 1 of the FDA guide we obtain the following human equivalent doses to the effective doses in rats to treat cancer (Targretin NDA #21055) and to regenerate neurons damaged by 60HDA (denoted PD in Table 8) when administered i.c.v. or the s.c. dose that delivers equivalent brain concentrations ofBexarotene to i.c.v. administration (see Table 4 above).

**Table 8.** Human equivalent dose using FDA guidelines

| | Effective dose | human equivalent dose | human total | dose |
|---|---|---|---|---|
| Therapeutic indication | rat | *(rat dose ÷ $K_m$) | s.c. or i.c.v. | oral |
| | mg/kg/day | mg/kg | mg/day | mg/day |
| Cancer | 60 | 9.7 | 774 | |
| PD s.c. | 0.25 | 0.040 | 3.2 | #4 to 43 |
| PD i.c.v. | 0.00625 | 0.001 | 0.08 | - |

*Used $K_m$ value of 6.2 taken from FDA guide, Table 1
#calculated assuming the oral bioavailability may range from 10.4 to 83.1°/ as was seen in dogs (Targretin NDA #21055)
Human total dose based on an 80 kg individual

As shown in Table 8, this method extrapolates the effective dose to treat cancer in rats (60 mg/kg/day) to 774 mg/day in humans, which is between the suggested starting dose of 648 mg/day and the optimum dose in many patients of 860 mg/day. Using the same extroplation method, the predicted effective doses to treat PD in humans are 3.2 mg/day (s.c. administration) and 0.08 mg/day (i.c.v. administration. Because we are comparing oral doses (human and rat cancer) to s.c. and i.c.v. doses (rat PD), in order to estimate effective oral PD doses in humans, we need to account for oral bioavailability. In dogs, the oral bioavailability ofBexarotene may range between 7.55 and 83.1% depending on the formulation used, and in rats the oral bioavailability is reported to be 22.7% for males and 47% for females (Targretin NDA #21055). Thus dividing the predicted s.c. dose of 3.2 mg/day by 0.0755 or 0.831 to account for the widest variation

gives an estimated range of human oral doses of 4 to 43 mg/day. These estimates are slightly lower, but generally in good agreement with estimates provided above in Table 7 and the narrative immediately following Table 7.

The invention should not be construed as limited to the dose ranges given in the examples. Dose ranges may also be affected by other factors such a patient compliance and individual patient response. Thus also dose ranges as used throughout the application are considered likely.

Although the invention has been described with reference to embodiments and examples, it should be understood that numerous and various modifications can be made without departing from the spirit of the invention. Accordingly, the invention is limited only by the following claims.

References

Carpentier, R., P. Sacchetti, et al. (2008). "The glucocorticoid receptor is a co-regulator of the orphan nuclear receptor Nurr1." J Neurochem 104(3): 777-89.

Castro, D.S., Arvidsson,M., Bondesson,B.M. and Perlmann,T. (1999) Activity of the Nurr1 carboxyl-terminal domain depends on cell type and integrity of the activation function 2. J. Biol. Chem., 274, 37483-37490.

Dubois, C., B. Hengerer, et al. (2006). "Identification of a potent agonist of the orphan nuclear receptor Nurr1." ChemMedChem 1(9): 955-8.

Duvic M, Hymes K, Heald P, Breneman D, Martin AG, Myskowski P, Crowley C, Yocum RC; Bexarotene Worldwide Study Group. Bexarotene is effective and safe for treatment of refractory advanced-stage cutaneous T-cell lymphoma: multinational phase II-III trial results. J Clin Oncol. 2001 May 1;19(9):2456-71.

FDA recommended methods to extrapolate between human and animal dosing data:

www.fda.gov/downloads/Drugs/GuidanceComplianceRegulatoryInformation/Guidan ces/ucm078932.pdf

Gniadecki R, Assaf C, Bagot M, Dummer R, Duvic M, Knobler R, Ranki A, Schwandt P, Whittaker S. The optimal use ofbexarotene in cutaneous T-cell lymphoma. Br J Dermatol. 2007 Sep;157(3):433-40.

Hou, J. G., Lin, L. F., & Mytilineou, C. (1996). Glial cell line-derived neurotrophic factor exerts neurotrophic effects on dopaminergic neurons in vitro and promotes their survival and regrowth after damage by 1-methyl-4-phenylpyridinium. J Neurochem., 66, 74-82.

Hung, H. C. & Lee, E. H. (1996). The mesolimbic dopaminergic pathway is more resistant than the nigrostriatal dopaminergic pathway to MPTP and MPP+ toxicity: role of BDNF gene expression. Brain Res Mol Brain Res., 41, 14-26.

Jankovic, J., S. Chen, et al. (2005). "The role of Nurr1 in the development of dopaminergic neurons and Parkinson's disease." Prog Neurobiol 77(1-2): 128-38.

Lund BW, Piu F, Gauthier NK, Eeg A, Currier E, Sherbukhin V, Brann MR, Hacksell U, Olsson R. Discovery of a potent, orally available, and isoform-selective retinoic acid beta2 receptor agonist. J Med Chem. 2005 Dec 1;48(24): 7517-9.

Hermanson, E., B. Joseph, et al. (2003). "Nurr1 regulates dopamine synthesis and storage in MN9D dopamine cells." Exp Cell Res 288(2): 324-34.

Miller VA, Benedetti FM, Rigas JR, Verret AL, Pfister DG, Straus D, Kris MG, Crisp M, Heyman R, Loewen GR, Truglia JA, Warrell RP Jr. (1997) Initial clinical trial of a selective retinoid X receptor ligand, LGD1069. J Clin Oncol. 15:790-795.

Ohta K, Kawachi E, Inoue N, Fukasawa H, Hashimoto Y, Itai A, Kagechika H. Retinoidal pyrimidinecarboxylic acids. Unexpected diaza-substituent effects in retinobenzoic acids. Chem Pharm Bull (Tokyo). 2000 Oct;48(10):1504-13.

Ordentlich, P., Y. Yan, et al. (2003). "Identification of the antineoplastic agent 6-mercaptopurine as an activator of the orphan nuclear hormone receptor Nurr1." J Biol Chem 278(27): 24791-9.

Paisan-Ruiz, C., Jain, S., Evans, E. W., Gilks, W. P., Simon, J., van der, B. M., Lopez, d. M., Aparicio, S., Gil, A. M., Khan, N., Johnson, J., Martinez, J. R., Nicholl, D., Carrera, I. M., Pena, A. S., de Silva, R., Lees, A., Marti-Masso, J. F., Perez-Tur, J., Wood, N. W., & Singleton, A. B. (2004). Cloning of the gene containing mutations that cause PARK8-linked Parkinson's disease. Neuron., 44, 595-600.

Sacchetti,P., Dwornik,H., Formstecher,P., Rachez,C. and Lefebvre,P. (2002) Requirements for heterodimerization between the orphan nuclear receptor nurr1 and retinoid X receptors. J. Biol. Chem., 277, 35088-35096.

Saijo, K., B. Winner, et al. (2009). "A Nurr1/CoREST pathway in microglia and astrocytes protects dopaminergic neurons from inflammation-induced death." Cell 137(1): 47-59.

Schinelli, S., Zuddas, A., Kopin, I. J., Barker, J. L., & DI Porzio, U. (1988). 1-Methyl-4-phenyl-1,2,3,6-tetrahydropyridine metabolism and 1-methyl-4- phenylpyridinium uptake in dissociated cell cultures from the embryonic mesencephalon. J Neurochem., 50, 1900-1907.
Shi, Y. (2007). "Orphan nuclear receptors in drug discovery." Drug Discov Today 12(11-12): 440-5.
Tan, P. K., J. Wang, et al. (2007). "Monitoring interactions between receptor tyrosine kinases and their downstream effector proteins in living cells using bioluminescence resonance energy transfer." Mol Pharmacol 72(6): 1440-6.
Targretin NDA #21055. Information may be found at http://www.accessdata.fda.gov/drugsatfda_docs/nda/99/21055_Targretin.cfm. See Pharmacology Review, Part 1.
Uhl, G. R., Javitch, J. A., & Snyder, S. H. (1985). Normal MPTP binding in parkinsonian substantial nigra: evidence for extraneuronal toxin conversion in human brain. Lancet., 1, 956-957.
Umemiya H, Fukasawa H, Ebisawa M, Eyrolles L, Kawachi E, Eisenmann G, Gronemeyer H, Hashimoto Y, Shudo K, Kagechika H. Regulation of retinoidal actions by diazepinylbenzoic acids. Retinoid synergists which activate the RXR-RAR heterodimers. J Med Chem. 1997 Dec 19;40(26):4222-34.
Vila, M. & Przedborski, S. (2004). Genetic clues to the pathogenesis of Parkinson's disease. Nat. Med., 10 Suppl, S58-S62.
Wagner CE, Jurutka PW, Marshall PA, Groy TL, van der Vaart A, Ziller JW, Furmick JK, Graeber ME, Matro E, Miguel BV, Tran IT, Kwon J, Tedeschi JN, Moosavi S, Danishyar A, Philp JS, Khamees RO, Jackson JN, Grupe DK, Badshah SL, Hart JW. Modeling, synthesis and biological evaluation of potential retinoid X receptor (RXR) selective agonists: novel analogues of 4-[1-(3,5,5,8,8-pentamethyl-5,6,7,8-tetrahydro-2-naphthyl)ethynyl]benzoic acid (bexarotene). J Med Chem. 2009 Oct 8;52(19):5950-66.
Wallen-Mackenzie, A., A. Mata de Urquiza, et al. (2003). "Nurr1-RXR heterodimers mediate RXR ligand-induced signaling in neuronal cells." Genes Dev 17(24): 3036-47.
Zetterstrom, R. H., L. Solomin, et al. (1997). "Dopamine neuronagenesis in Nurr1-deficient mice." Science 276 (5310): 248-50.

## Claims

1. A compound of formula (I)

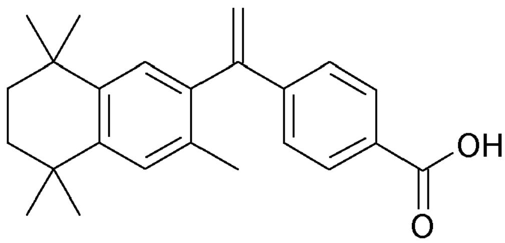

(I)

or a pharmaceutically acceptable salt, solvate, polymorph or hydrate thereof, for use in the treatment of a neurodegenerative disease or disorder wherein said compound is to be administered in a low dose.

2. A compound for use according to claim 1, wherein said compound is to be administered in a dose of 0.05-75 mg per day.

3. A compound for use according to claim 1 or claim 2, wherein said compound is to be administered in a dose of 0.05-65 mg per day or in a dose of 0.05-50 mg per day.

4. A compound for use according to any one of the claims 1-3, wherein said compound is to be administered orally.

5. A compound for use according to any one of the claims 1-3, wherein said compound is to be administered through a non-oral route of administration.

6. A compound for use according to claim 5, wherein said compound is to be administered sub-cutaneously or transdermally.

7. A compound for use according to claim 5, wherein said compound is to be administered intracerebroventricullarly.

8. A compound for use according to claim 7, wherein said compound is to be administered in a dose of 0.05-20 mg

per day.

**9.** A compound for use according to claim 7 or claim 8, wherein said compound is to be administered in a dose of 0.05-15 mg per day.

**10.** A compound for use according to any one of the claims 5-9, wherein said compound is to be administered in a continuous infusion.

**11.** A compound for use according to any one of the claims 1-10, wherein said neurodegenerative disease or disorder is a disease or disorder that benefits from regeneration of the function of dopaminergic neurons.

**12.** A compound for use according to any one of the claims 1-11, wherein said neurodegenerative disease is associated with a Nurr1 receptor.

**13.** A compound for use according to any one of the claims 1-12, wherein said neurodegenerative disease is Parkinson’s disease.

**14.** A compound for use according to any one of the claims 1-13, wherein administration of said compound to a patient will lead to regeneration of the function of dopaminergic neurons.

**15.** A compound for use according to claim 14, wherein the function of the dopaminergic neurons has been impaired as a result of Parkinson’s disease.

| | |
|---|---|
| Changes in intermolecular arrangement/dimerization: | Nurr1-GFP2 + RXRα-Rluc |
| | Nurr1-Rluc + Nurr1-GFP2 |
| | RXRα-GFP2 + RXRα-Rluc |
| Changes in intramolecular structure/conformational changes: | Rluc-RXRα-GFP |
| | Rluc-Nurr1-GFP |

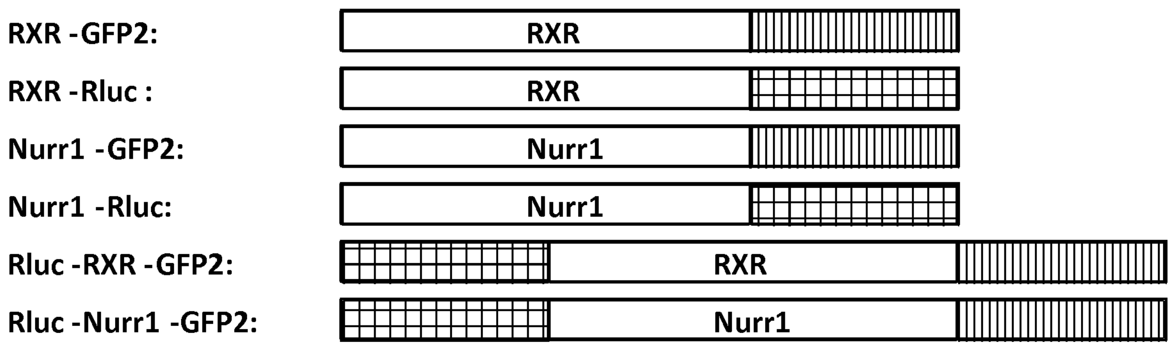


*Fig. 1*

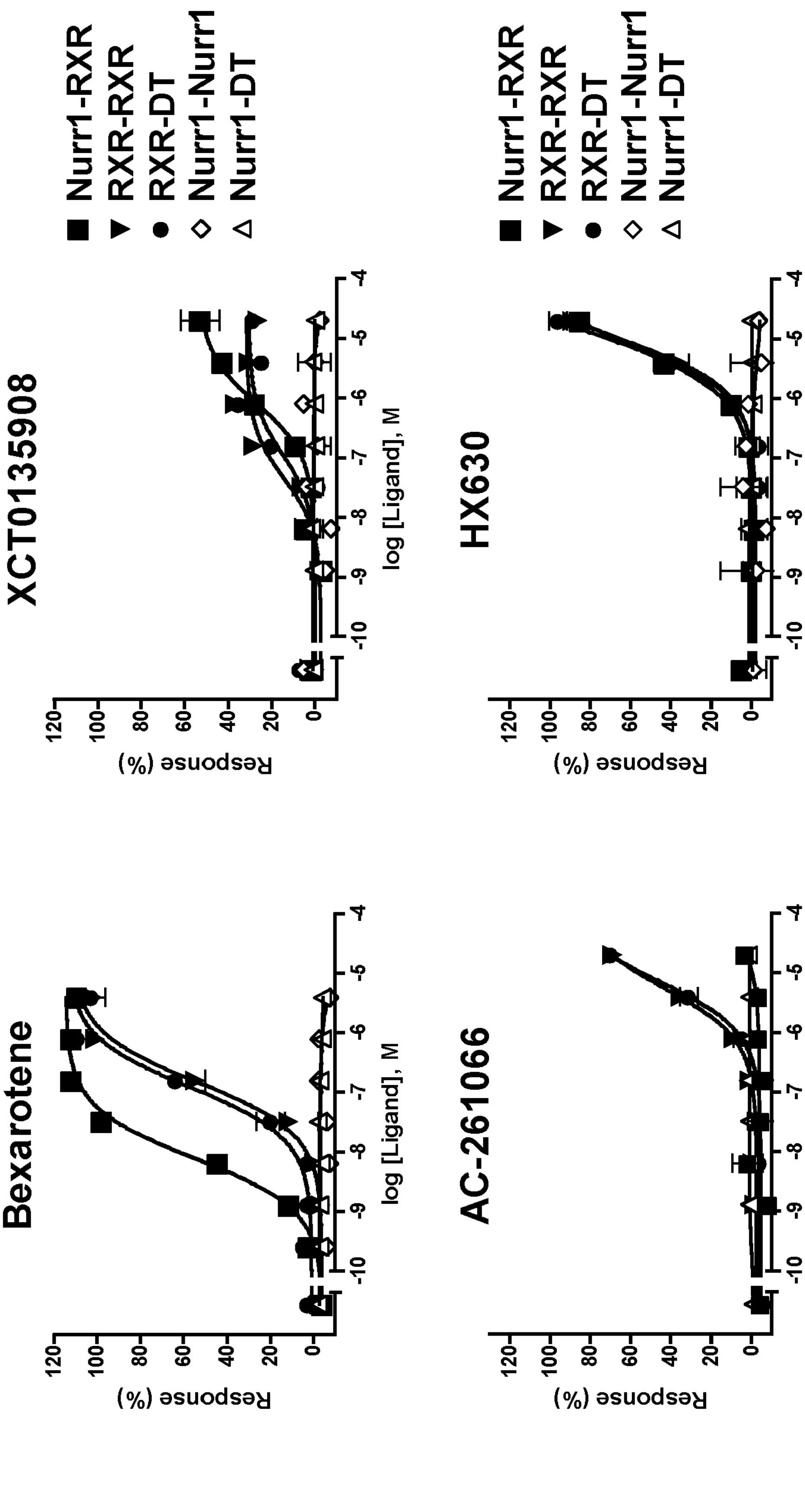

Bexarotene
XCT0135908
AC-261066
HX630
Response (%)
120
100
80
60
40
20
0
-10
-9
-8
-7
-6
-5
-4
log [Ligand], M
Nurr1-RXR
RXR-RXR
RXR-DT
Nurr1-Nurr1
Nurr1-DT


*Fig. 2*

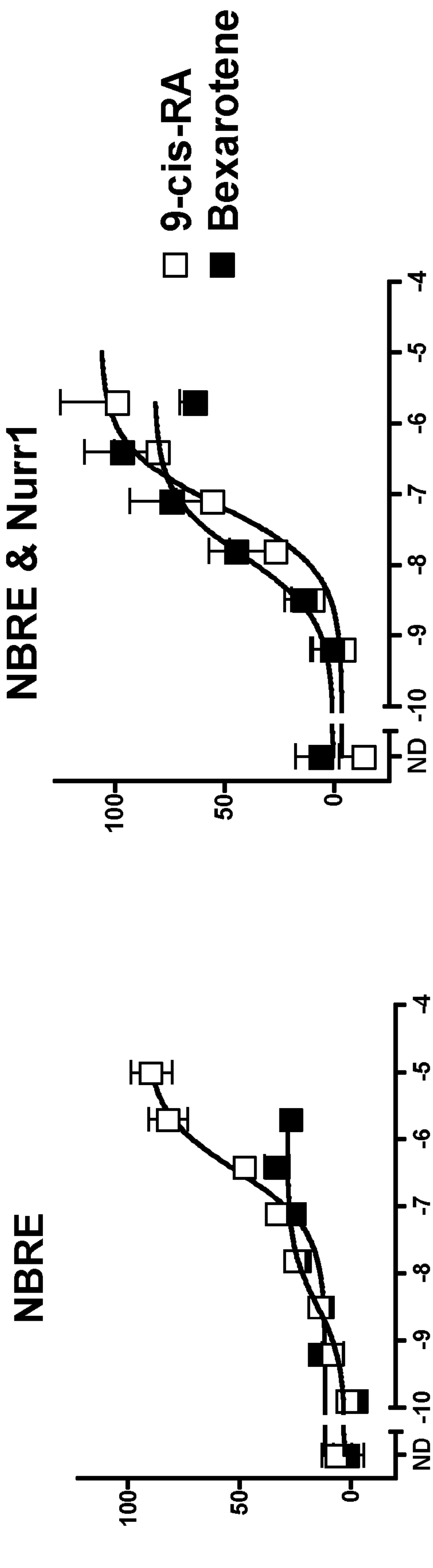
NBRE & Nurr1
9-cis-RA
Bexarotene
log [Ligand], M
ND -10 -9 -8 -7 -6 -5 -4
100
50
0
NBRE
log [Ligand], M
ND -10 -9 -8 -7 -6 -5 -4
100
50
0

*Fig. 3*

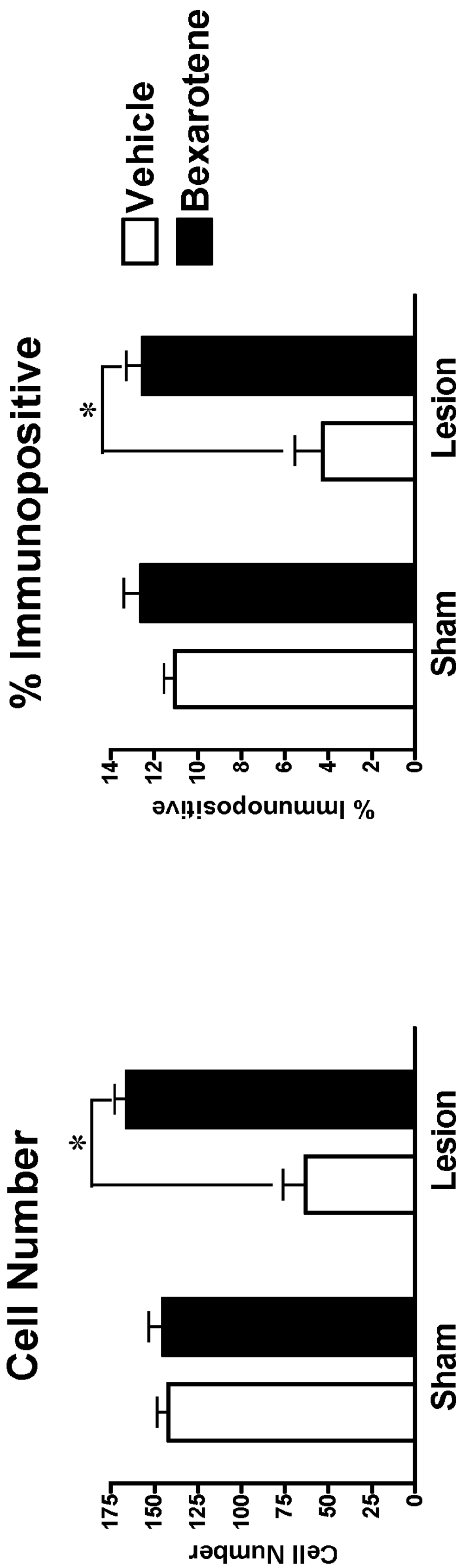
Cell Number
Cell Number
175
150
125
100
75
50
25
0
Sham
Lesion
*
% Immunopositive
% Immunopositive
14
12
10
8
6
4
2
0
Sham
Lesion
*
Vehicle
Bexarotene

*Fig. 4*

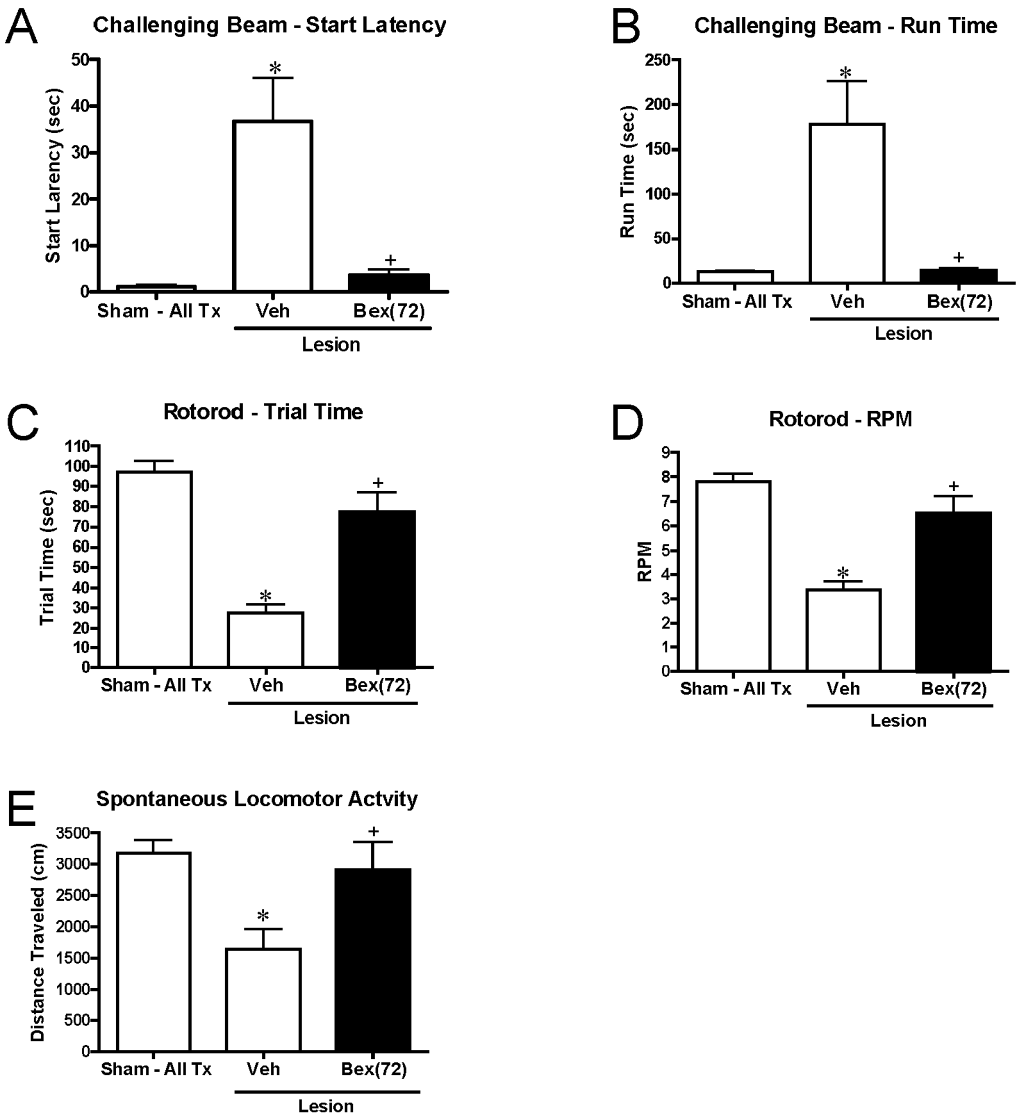
A
Challenging Beam - Start Latency
Start Larency (sec)
0
10
20
30
40
50
*
+
Sham - All Tx
Veh
Bex(72)
Lesion
B
Challenging Beam - Run Time
Run Time (sec)
0
50
100
150
200
250
*
+
Sham - All Tx
Veh
Bex(72)
Lesion
C
Rotorod - Trial Time
Trial Time (sec)
0
10
20
30
40
50
60
70
80
90
100
110
*
+
Sham - All Tx
Veh
Bex(72)
Lesion
D
Rotorod - RPM
RPM
0
1
2
3
4
5
6
7
8
9
*
+
Sham - All Tx
Veh
Bex(72)
Lesion
E
Spontaneous Locomotor Actvity
Distance Traveled (cm)
0
500
1000
1500
2000
2500
3000
3500
*
+
Sham - All Tx
Veh
Bex(72)
Lesion

*Fig. 5*

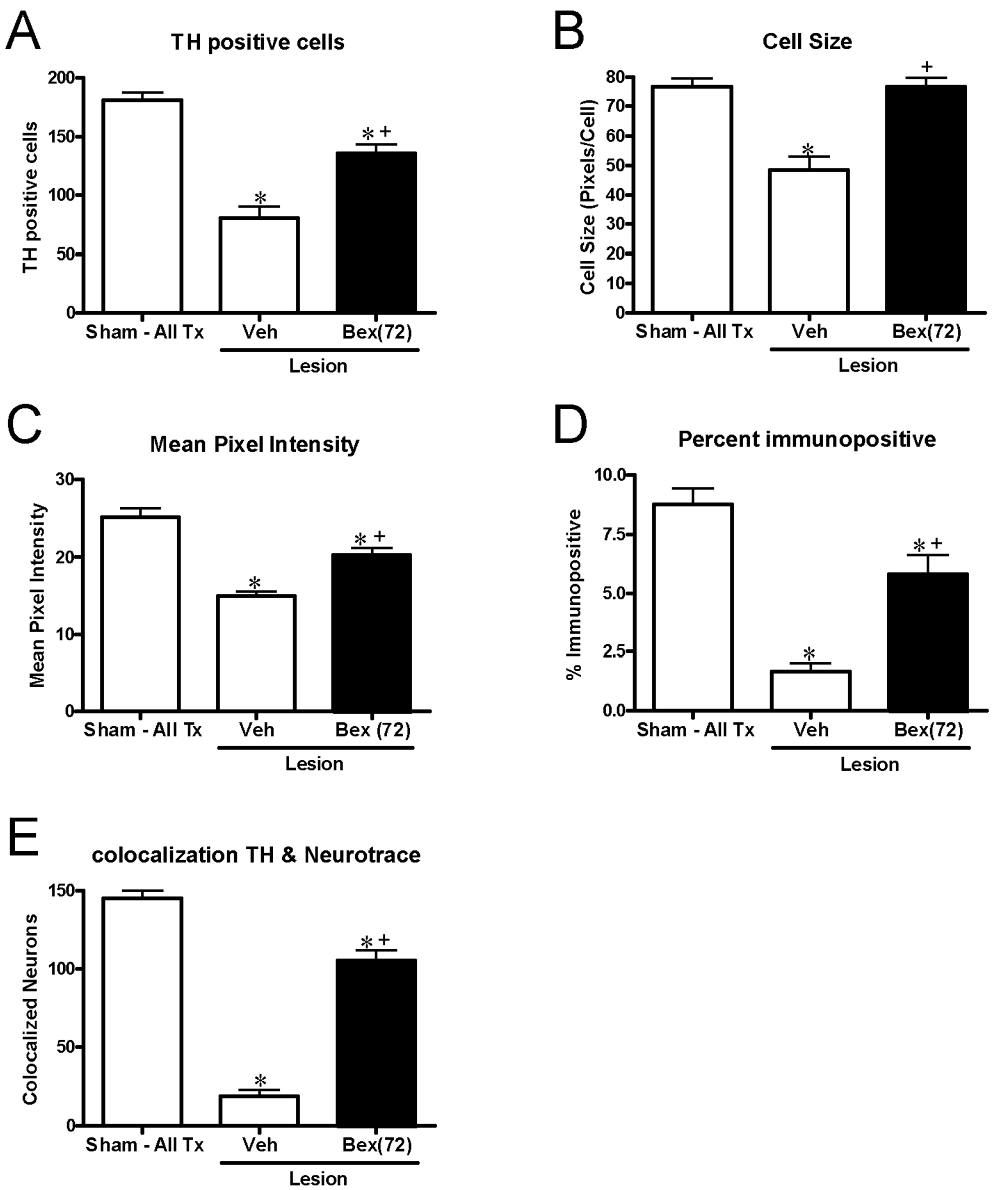
A
TH positive cells
TH positive cells
200
150
100
50
0
Sham - All Tx
Veh
Bex(72)
Lesion
B
Cell Size
Cell Size (Pixels/Cell)
80
70
60
50
40
30
20
10
0
Sham - All Tx
Veh
Bex(72)
Lesion
C
Mean Pixel Intensity
Mean Pixel Intensity
30
20
10
0
Sham - All Tx
Veh
Bex (72)
Lesion
D
Percent immunopositive
% Immunopositive
10.0
7.5
5.0
2.5
0.0
Sham - All Tx
Veh
Bex(72)
Lesion
E
colocalization TH & Neurotrace
Colocalized Neurons
150
100
50
0
Sham - All Tx
Veh
Bex(72)
Lesion

*Fig. 6*

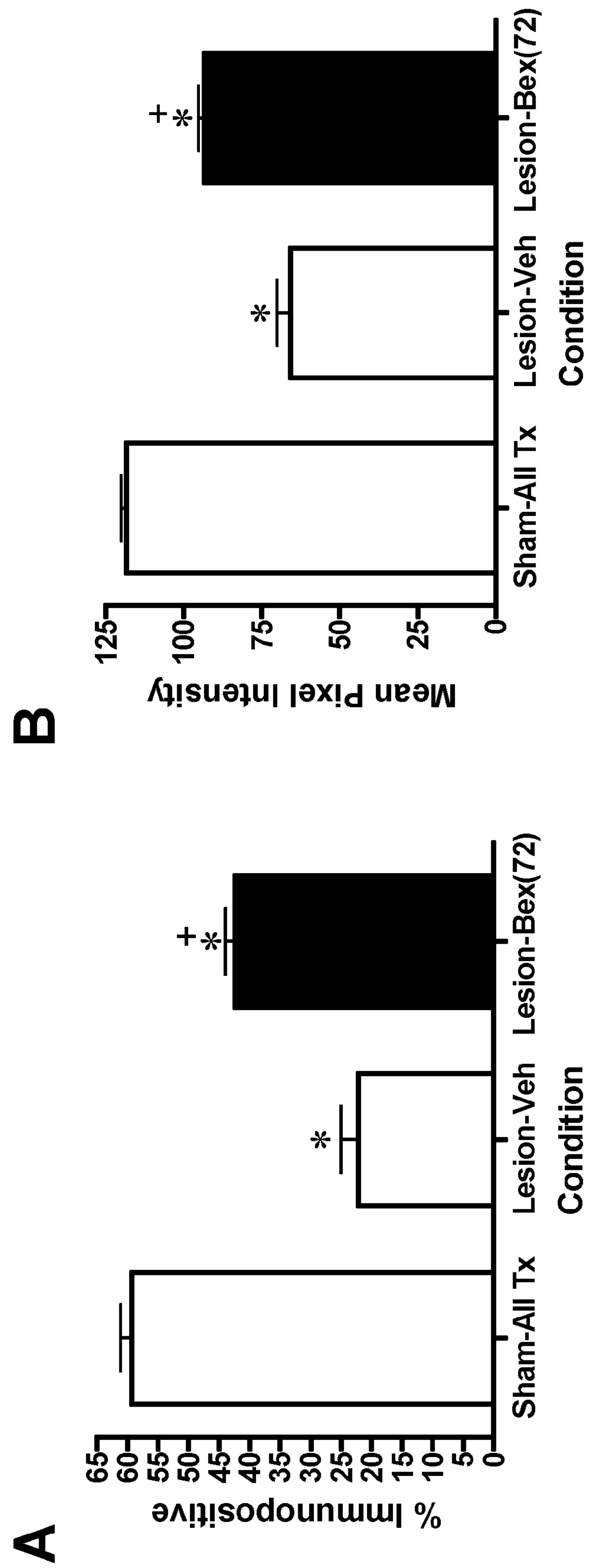
A
% Immunopositive
0
5
10
15
20
25
30
35
40
45
50
55
60
65
Sham-All Tx
Lesion-Veh
Lesion-Bex(72)
Condition
B
Mean Pixel Intensity
0
25
50
75
100
125
Sham-All Tx
Lesion-Veh
Lesion-Bex(72)
Condition

*Fig. 7*

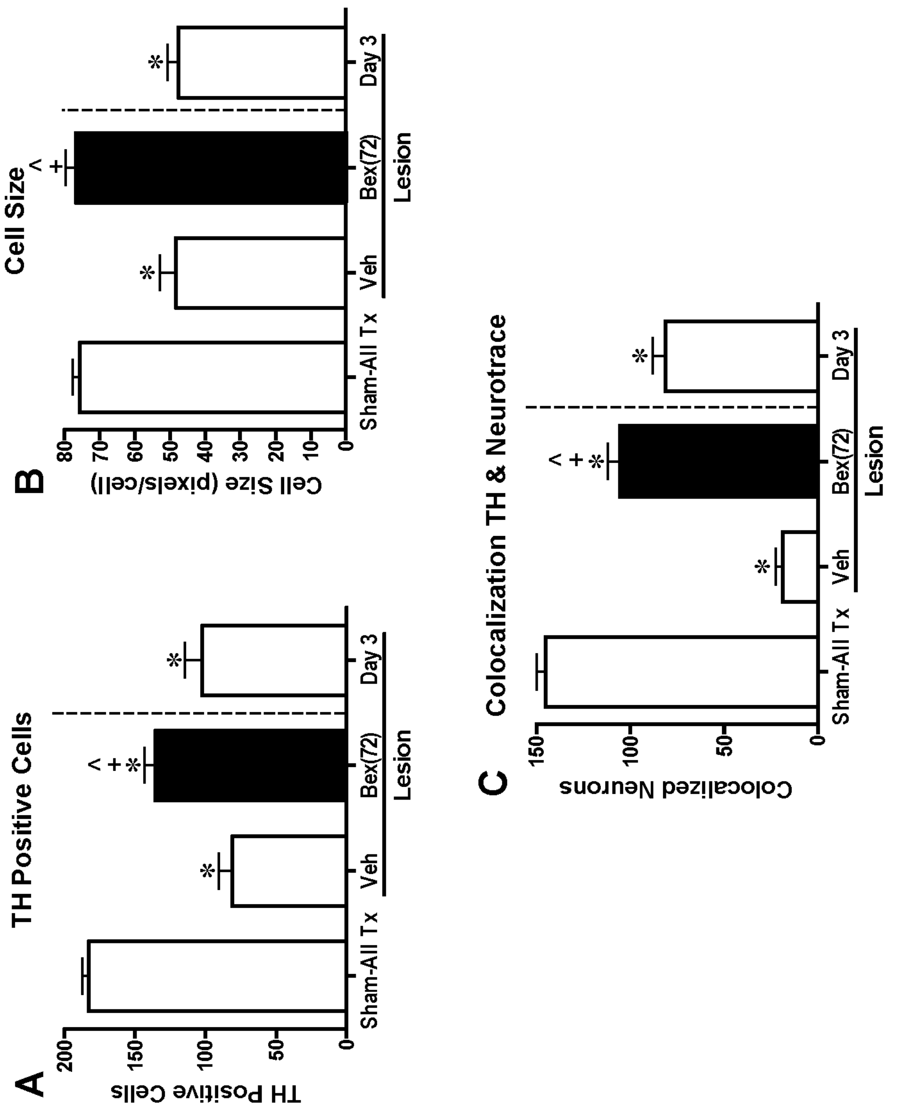
A
TH Positive Cells
TH Positive Cells
200
150
100
50
0
Sham-All Tx
Veh
Bex(72)
Day 3
Lesion
B
Cell Size
Cell Size (pixels/cell)
80
70
60
50
40
30
20
10
0
Sham-All Tx
Veh
Bex(72)
Day 3
Lesion
C
Colocalization TH & Neurotrace
Colocalized Neurons
150
100
50
0
Sham-All Tx
Veh
Bex(72)
Day 3
Lesion

*Fig. 8*

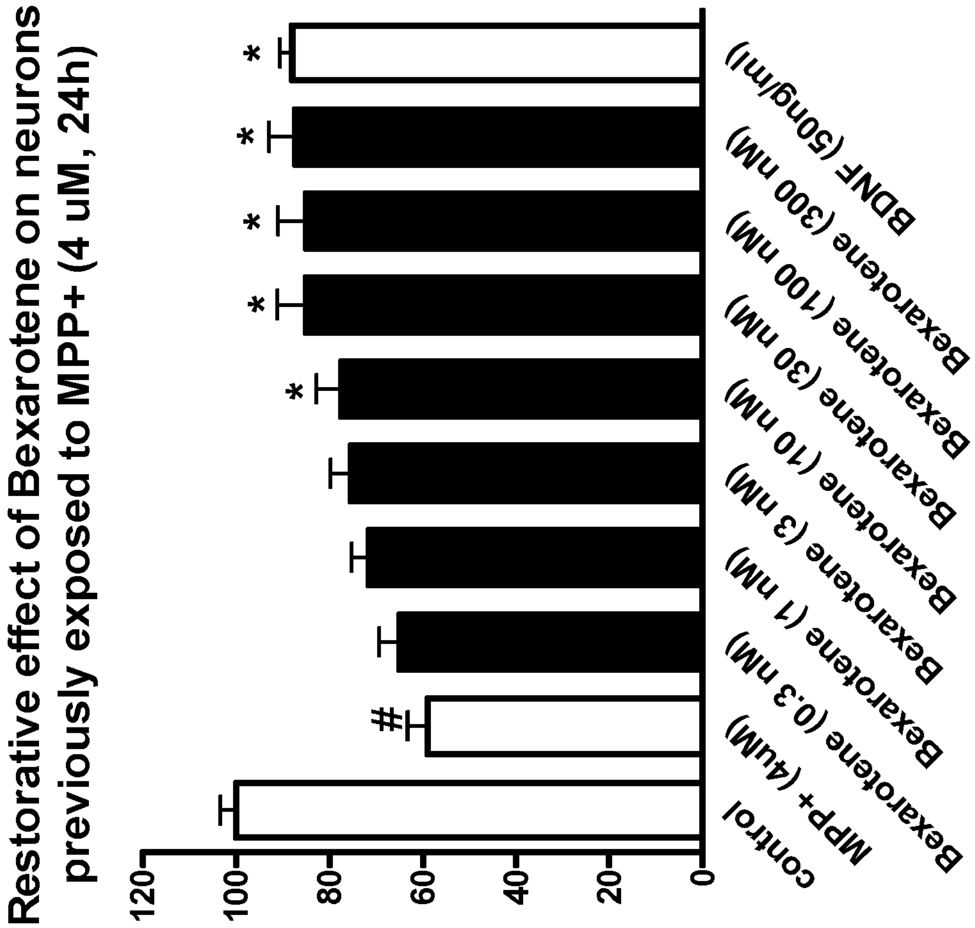
Restorative effect of Bexarotene on neurons previously exposed to MPP+ (4 uM, 24h)
TH positive neurons (%)
0
20
40
60
80
100
120
control
MPP+ (4uM)
Bexarotene (0.3 nM)
Bexarotene (1 nM)
Bexarotene (3 nM)
Bexarotene (10 nM)
Bexarotene (30 nM)
Bexarotene (100 nM)
Bexarotene (300 nM)
BDNF (50ng/ml)
#
*
*
*
*
*

*Fig. 9 A*

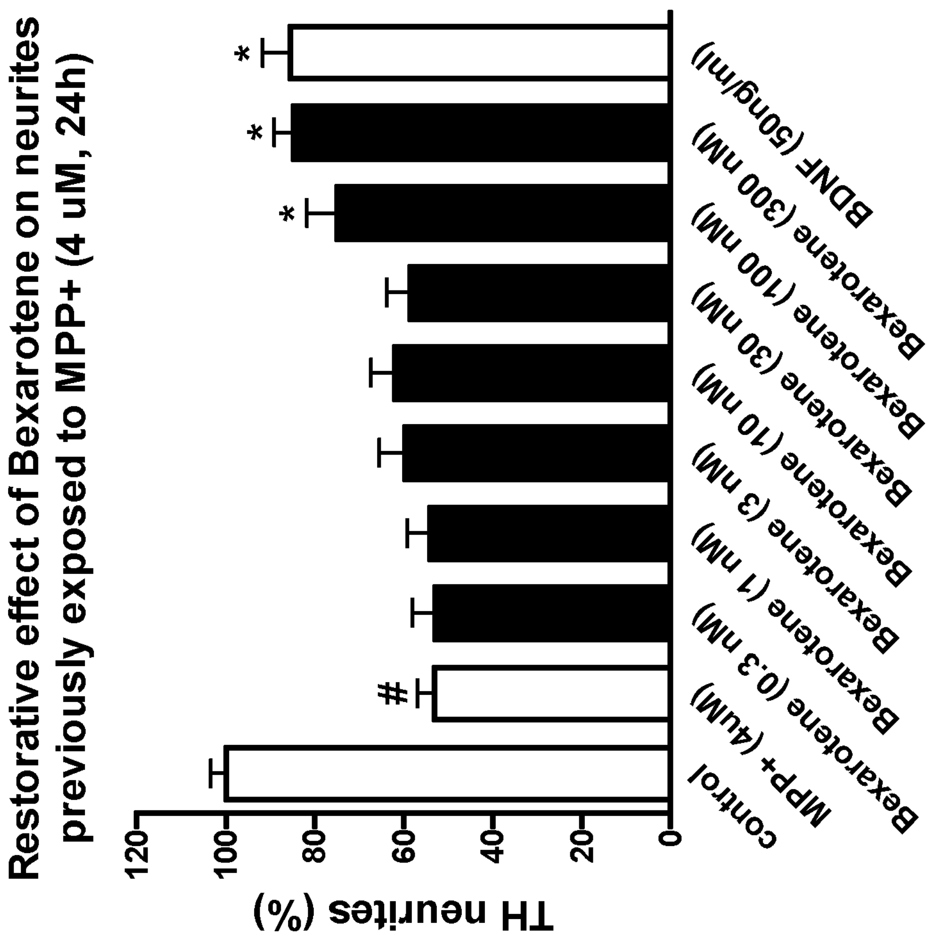
Restorative effect of Bexarotene on neurites
previously exposed to MPP+ (4 uM, 24h)
TH neurites (%)
0
20
40
60
80
100
120
#
*
*
*
control
MPP+ (4uM)
Bexarotene (0.3 nM)
Bexarotene (1 nM)
Bexarotene (3 nM)
Bexarotene (10 nM)
Bexarotene (30 nM)
Bexarotene (100 nM)
Bexarotene (300 nM)
BDNF (50ng/ml)

*Fig. 9 B*

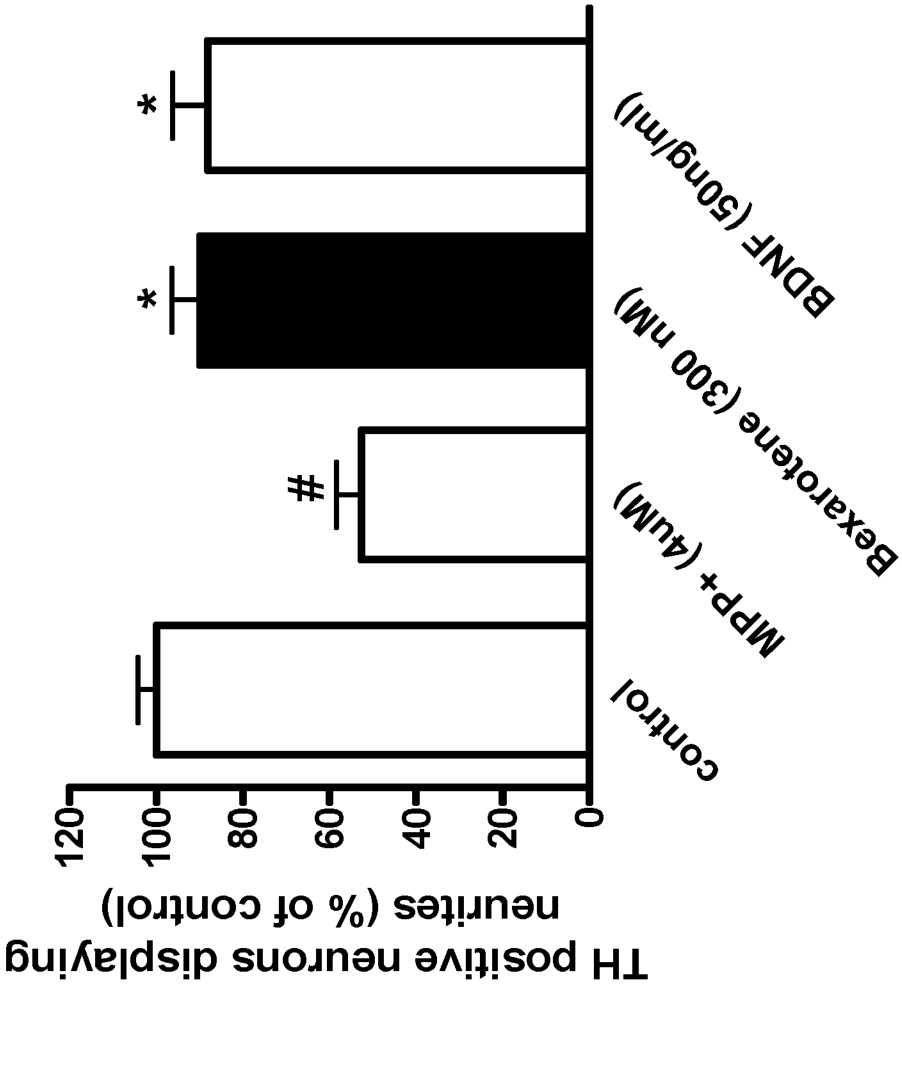
TH positive neurons displaying neurites (% of control)
0
20
40
60
80
100
120
control
MPP+ (4uM)
Bexarotene (300 nM)
BDNF (50ng/ml)
#
*
*

*Fig. 9 C*

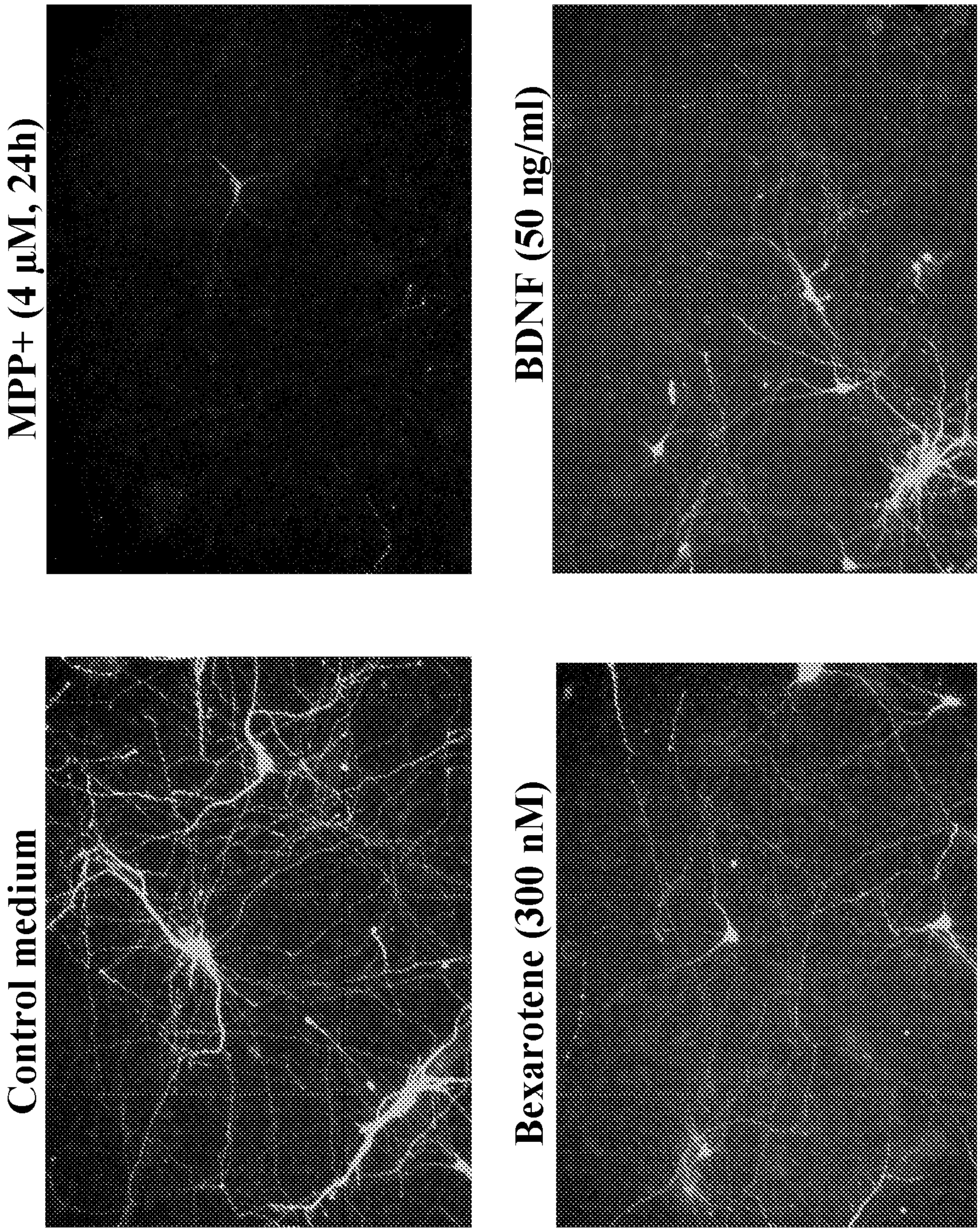


*Fig. 10*

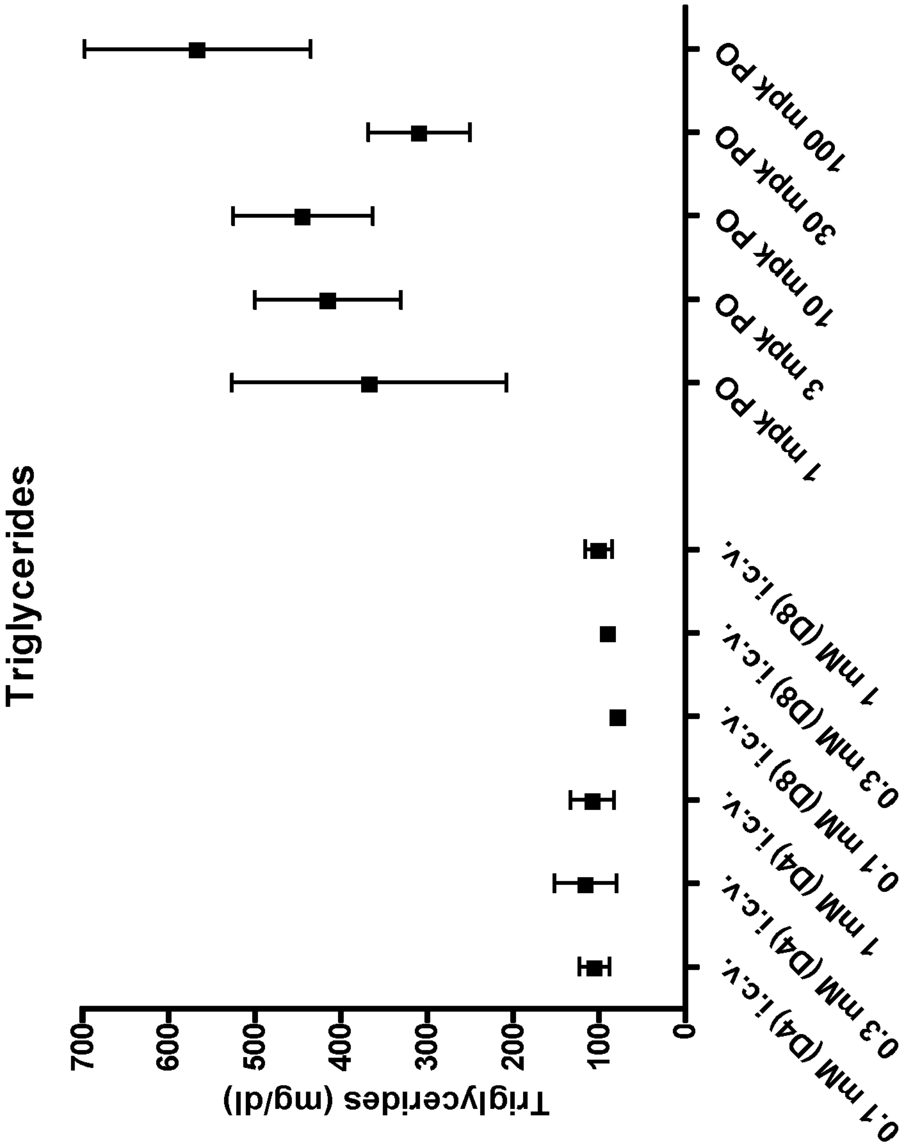
Triglycerides
Triglycerides (mg/dl)
0
100
200
300
400
500
600
700
0.1 mM (D4) i.c.v.
0.3 mM (D4) i.c.v.
1 mM (D4) i.c.v.
0.1 mM (D8) i.c.v.
0.3 mM (D8) i.c.v.
1 mM (D8) i.c.v.
1 mpk PO
3 mpk PO
10 mpk PO
30 mpk PO
100 mpk PO

*Fig. 11*

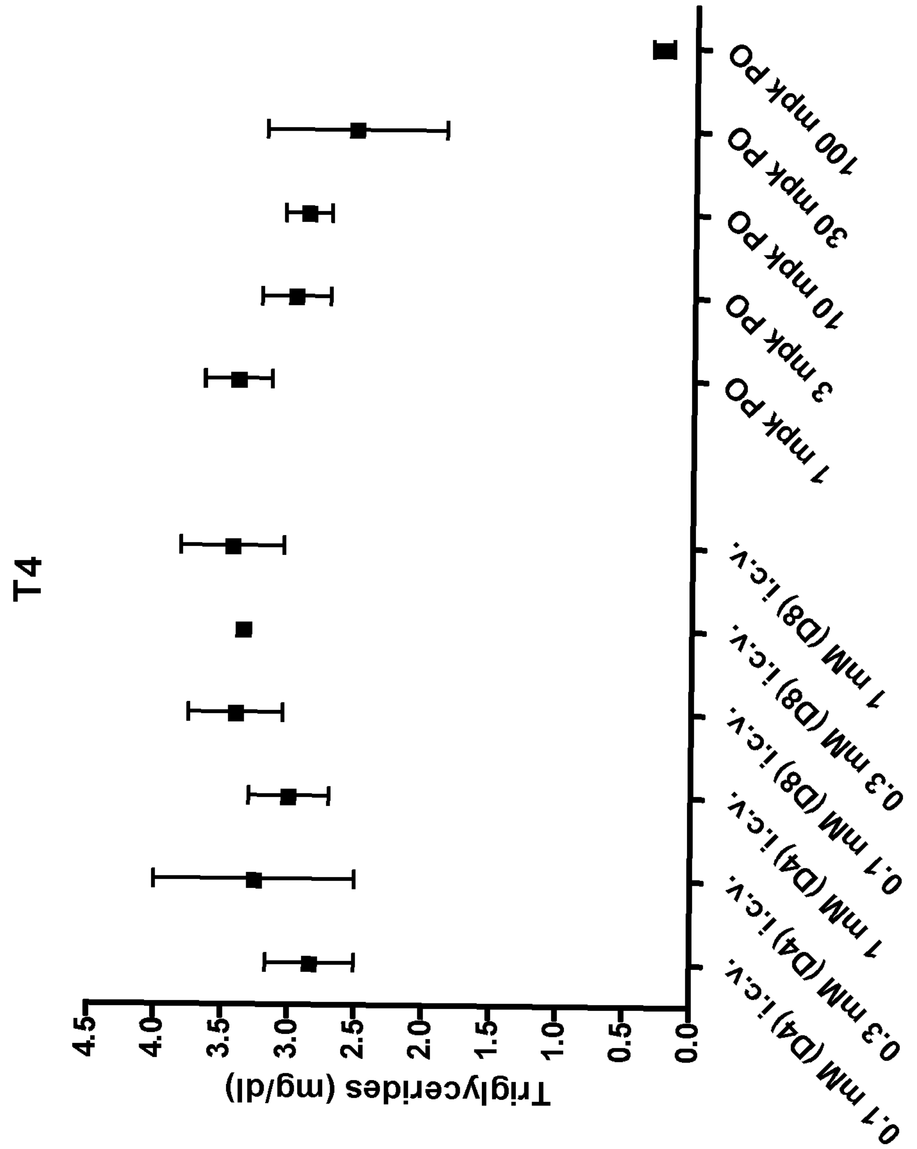
T4
Triglycerides (mg/dl)
0.0
0.5
1.0
1.5
2.0
2.5
3.0
3.5
4.0
4.5
0.1 mM (D4) i.c.v.
0.3 mM (D4) i.c.v.
1 mM (D4) i.c.v.
0.1 mM (D8) i.c.v.
0.3 mM (D8) i.c.v.
1 mM (D8) i.c.v.
1 mpk PO
3 mpk PO
10 mpk PO
30 mpk PO
100 mpk PO

*Fig. 12*

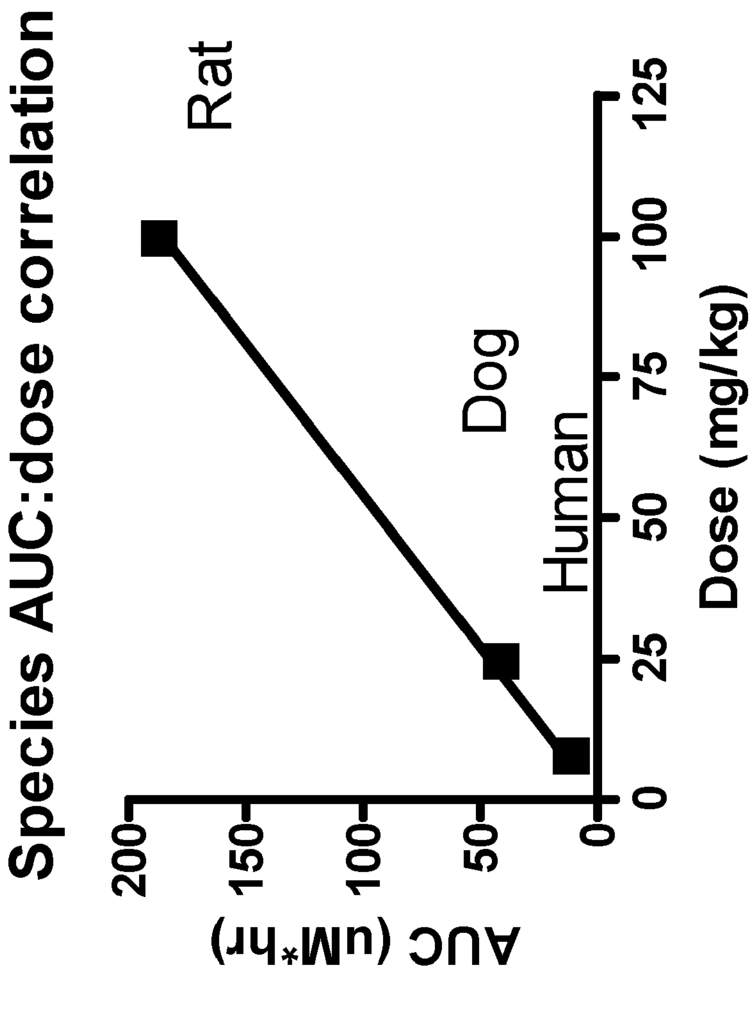
Species AUC:dose correlation
AUC (uM*hr)
200
150
100
50
0
0
25
50
75
100
125
Dose (mg/kg)
Rat
Dog
Human

*Fig. 13*

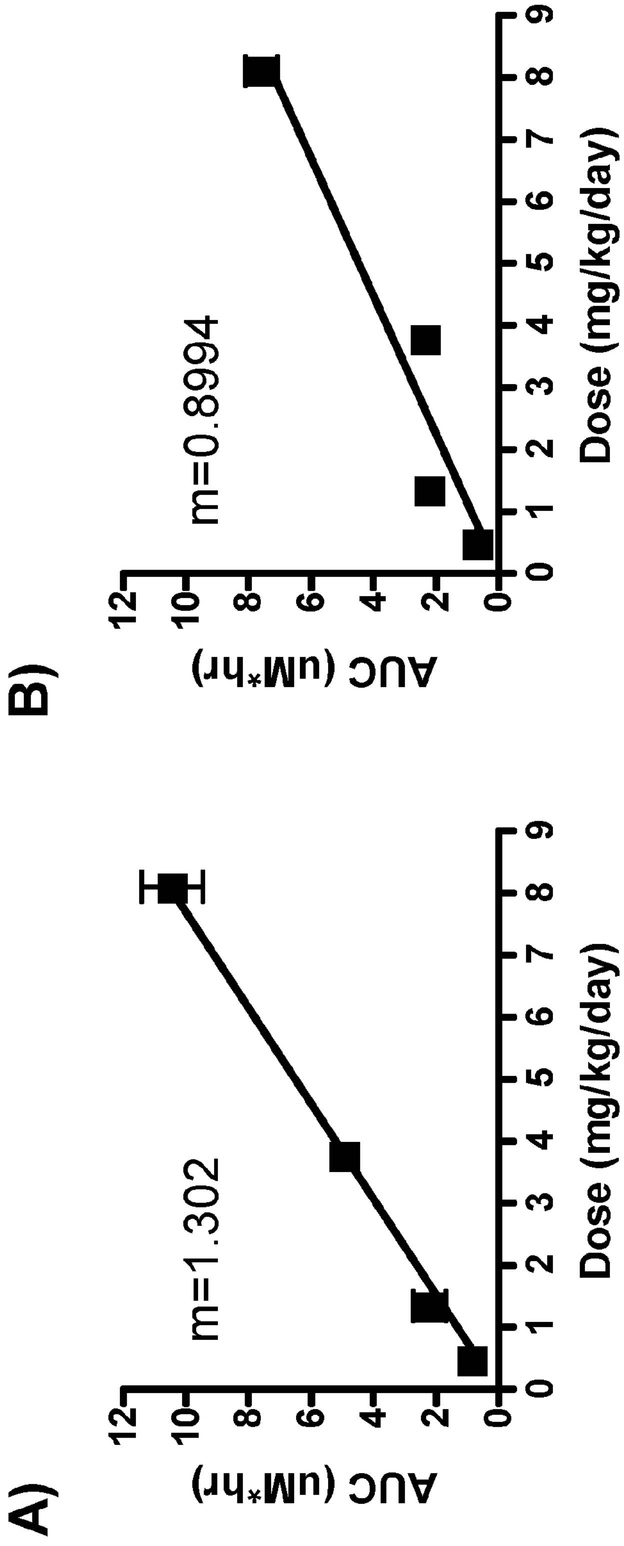
A)
AUC (uM*hr)
0
2
4
6
8
10
12
m=1.302
Dose (mg/kg/day)
0
1
2
3
4
5
6
7
8
9
B)
AUC (uM*hr)
0
2
4
6
8
10
12
m=0.8994
Dose (mg/kg/day)
0
1
2
3
4
5
6
7
8
9

*Fig. 14*

Europäisches Patentamt
European Patent Office
Office européen des brevets

# EUROPEAN SEARCH REPORT

Application Number
EP 11 17 7210

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | WO 2011/057022 A1 (PLEXXIKON INC [US]; ZHANG JIAZHONG [US]; IBRAHIM PRABHA N [US]; BREMER) 12 May 2011 (2011-05-12) * paragraph [0116] - paragraph [0198]; claims 1, 40-47 * ----- | 1-15 | INV. A61K31/192 A61P25/16 C07C49/792 |
| Y | WO 2009/146218 A2 (REATA PHARMACEUTICALS INC [US]; ANDERSON ERIC [US]; BOLTON GARY L [US]) 3 December 2009 (2009-12-03) * page 25 - page 75; claims 1,110, 133-136 * ----- | 1-15 | |
| Y | WO 2009/146216 A2 (REATA PHARMACEUTICALS INC [US]; JIANG XIN [US]; GREINER JACK; MARAVETZ) 3 December 2009 (2009-12-03) * page 14 - page 17; claims 1,138, 161-164 * ----- | 1-15 | |
| Y | WO 2008/064133 A1 (DARTMOUTH COLLEGE [US]; HONDA TADASHII [US]; SUNDARARAJAN CHITRA; GRIB) 29 May 2008 (2008-05-29) * page 19 - page 33; claims 1,104, 152, 153 * ----- | 1-15 | TECHNICAL FIELDS SEARCHED (IPC): A61K A61P C07C |

The present search report has been drawn up for all claims

1

| Place of search | Date of completion of the search | Examiner |
|---|---|---|
| Munich | 23 December 2011 | Kling, Isabelle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 11 17 7210

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2011

| Patent document cited in search report | Publication date | Patent family member(s) | Publication date |
|---|---|---|---|
| WO 2011057022 A1 | 12-05-2011 | TW 201121969 A | 01-07-2011 |
| | | US 2011112127 A1 | 12-05-2011 |
| | | UY 33016 A | 31-05-2011 |
| | | WO 2011057022 A1 | 12-05-2011 |
| WO 2009146218 A2 | 03-12-2009 | AU 2009251437 A1 | 03-12-2009 |
| | | CA 2721665 A1 | 03-12-2009 |
| | | CN 102066397 A | 18-05-2011 |
| | | CO 6311105 A2 | 22-08-2011 |
| | | EA 201001556 A1 | 30-06-2011 |
| | | EP 2279196 A2 | 02-02-2011 |
| | | JP 2011518193 A | 23-06-2011 |
| | | KR 20110010609 A | 01-02-2011 |
| | | TW 201006474 A | 16-02-2010 |
| | | US 2010048887 A1 | 25-02-2010 |
| | | WO 2009146218 A2 | 03-12-2009 |
| WO 2009146216 A2 | 03-12-2009 | TW 201004627 A | 01-02-2010 |
| | | US 2010048911 A1 | 25-02-2010 |
| | | WO 2009146216 A2 | 03-12-2009 |
| WO 2008064133 A1 | 29-05-2008 | CA 2670099 A1 | 29-05-2008 |
| | | EP 2094651 A1 | 02-09-2009 |
| | | JP 2010510243 A | 02-04-2010 |
| | | US 2008261985 A1 | 23-10-2008 |
| | | US 2011009363 A1 | 13-01-2011 |
| | | WO 2008064133 A1 | 29-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. Mack Publishing Co, 1990
- **SHEDDEN et al.** *Clin. Ther.,* 2001, vol. 23 (3), 440-50
- **MAYER et al.** *Ophthalmologica,* 1996, vol. 210 (2), 101-3
- **JOSHI, A.** *J. Ocul. Pharmacol.,* 1994, vol. 10 (1), 29-45
- **ALM et al.** *Prog. Clin. Biol. Res.,* 1989, vol. 312, 447-58
- **MORDENTI.** *Toxicol. Sci.,* 1999, vol. 52 (1), 101-6
- **FINGL et al.** *The Pharmacological Basis of Therapeutics,* 1975
- **MILLER et al.** *J. Clin. Oncol.,* 1997
- **BOEHM et al.** *J. Med. Chem.,* 1994
- **BOEHM et al.** *J. Med. Chem.,* 1995
- **WALLEN-MACKENZIE et al.** *Genes Dev.,* 2003
- **UMEMIYA et al.** *J. Med. Chem.,* 1997
- **OHTA et al.** *J. Med. Chem.,* 2000
- **LUND et al.** *J. Med. Chem.,* 2005
- **DUBOIS et al.** *Chem. Med. Chem.,* 2006
- **ORDENTLICH et al.** *J. Biol. Chem.,* 2003
- **DUVIC et al.** *J. Clin. Oncol.,* 2001
- **DUVIC et al.** *J Clin Oncol,* 2001
- **MILLER et al.** *J Clin Oncol,* 1997
- **CARPENTIER, R. ; P. SACCHETTI et al.** The glucocorticoid receptor is a co-regulator of the orphan nuclear receptor Nurr1. *J Neurochem,* 2008, vol. 104 (3), 777-89
- **CASTRO, D.S. ; ARVIDSSON,M. ; BONDESSON, B.M. ; PERLMANN,T.** Activity of the Nurr1 carboxyl-terminal domain depends on cell type and integrity of the activation function 2. *J. Biol. Chem.,* 1999, vol. 274, 37483-37490
- **DUBOIS, C. ; B. HENGERER et al.** Identification of a potent agonist of the orphan nuclear receptor Nurr1. *ChemMedChem,* 2006, vol. 1 (9), 955-8
- **DUVIC M ; HYMES K ; HEALD P ; BRENEMAN D ; MARTIN AG ; MYSKOWSKI P ; CROWLEY C ; YOCUM RC.** Bexarotene Worldwide Study Group. Bexarotene is effective and safe for treatment of refractory advanced-stage cutaneous T-cell lymphoma: multinational phase II-III trial results. *J Clin Oncol.,* 01 May 2001, vol. 19 (9), 2456-71
- **GNIADECKI R ; ASSAF C ; BAGOT M ; DUMMER R ; DUVIC M ; KNOBLER R ; RANKI A ; SCHWANDT P ; WHITTAKER S.** The optimal use ofbexarotene in cutaneous T-cell lymphoma. *Br J Dermatol.,* September 2007, vol. 157 (3), 433-40
- **HOU, J. G. ; LIN, L. F. ; MYTILINEOU, C.** Glial cell line-derived neurotrophic factor exerts neurotrophic effects on dopaminergic neurons in vitro and promotes their survival and regrowth after damage by 1-methyl-4-phenylpyridinium. *J Neurochem.,* 1996, vol. 66, 74-82
- **HUNG, H. C. ; LEE, E. H.** The mesolimbic dopaminergic pathway is more resistant than the nigrostriatal dopaminergic pathway to MPTP and MPP+ toxicity: role of BDNF gene expression. *Brain Res Mol Brain Res.,* 1996, vol. 41, 14-26
- **JANKOVIC, J. ; S. CHEN et al.** The role of Nurr1 in the development of dopaminergic neurons and Parkinson's disease. *Prog Neurobiol,* vol. 77 (1-2), 128-38
- **LUND BW ; PIU F ; GAUTHIER NK ; EEG A ; CURRIER E ; SHERBUKHIN V ; BRANN MR ; HACKSELL U ; OLSSON R.** Discovery of a potent, orally available, and isoform-selective retinoic acid beta2 receptor agonist. *J Med Chem.,* 01 December 2005, vol. 48 (24), 7517-9
- **HERMANSON, E. ; B. JOSEPH et al.** Nurr1 regulates dopamine synthesis and storage in MN9D dopamine cells. *Exp Cell Res,* 2003, vol. 288 (2), 324-34
- **MILLER VA ; BENEDETTI FM ; RIGAS JR ; VERRET AL ; PFISTER DG ; STRAUS D ; KRIS MG ; CRISP M ; HEYMAN R ; LOEWEN GR.** Initial clinical trial of a selective retinoid X receptor ligand, LGD1069. *J Clin Oncol.,* 2007, vol. 15, 790-795
- **OHTA K ; KAWACHI E ; INOUE N ; FUKASAWA H ; HASHIMOTO Y ; ITAI A ; KAGECHIKA H.** Retinoidal pyrimidinecarboxylic acids. Unexpected diaza-substituent effects in retinobenzoic acids. *Chem Pharm Bull (Tokyo,* October 2000, vol. 48 (10), 1504-13
- **ORDENTLICH, P. ; Y. YAN et al.** Identification of the antineoplastic agent 6-mercaptopurine as an activator of the orphan nuclear hormone receptor Nurr1. *J Biol Chem,* 2003, vol. 278 (27), 24791-9

- **PAISAN-RUIZ, C. ; JAIN, S. ; EVANS, E. W. ; GILKS, W. P. ; SIMON, J. ; VAN DER, B. M. ; LOPEZ, D. M. ; APARICIO, S. ; GIL, A. M. ; KHAN, N.** Cloning of the gene containing mutations that cause PARK8-linked Parkinson's disease. *Neuron.,* 2004, vol. 44, 595-600
- **SACCHETTI,P. ; DWORNIK,H. ; FORMSTECHER, P. ; RACHEZ,C. ; LEFEBVRE,P.** Requirements for heterodimerization between the orphan nuclear receptor nurr1 and retinoid X receptors. *J. Biol. Chem.,* 2002, vol. 277, 35088-35096
- **SAIJO, K. ; B. WINNER et al.** A Nurr1/CoREST pathway in microglia and astrocytes protects dopaminergic neurons from inflammation-induced death. *Cell,* 2009, vol. 137 (1), 47-59
- **SCHINELLI, S. ; ZUDDAS, A. ; KOPIN, I. J. ; BARKER, J. L. ; DI PORZIO, U.** 1-Methyl-4-phenyl-1,2,3,6-tetrahydropyridine metabolism and 1-methyl-4- phenylpyridinium uptake in dissociated cell cultures from the embryonic mesencephalon. *J Neurochem.,* 1988, vol. 50, 1900-1907
- **SHI, Y.** Orphan nuclear receptors in drug discovery. *Drug Discov Today,* 2007, vol. 12 (11-12), 440-5
- **TAN, P. K. ; J. WANG et al.** Monitoring interactions between receptor tyrosine kinases and their downstream effector proteins in living cells using bioluminescence resonance energy transfer. *Mol Pharmacol,* 2007, vol. 72 (6), 1440-6
- **UHL, G. R. ; JAVITCH, J. A. ; SNYDER, S. H.** Normal MPTP binding in parkinsonian substantial nigra: evidence for extraneuronal toxin conversion in human brain. *Lancet.,* 1985, vol. 1, 956-957
- **UMEMIYA H ; FUKASAWA H ; EBISAWA M ; EYROLLES L ; KAWACHI E ; EISENMANN G ; GRONEMEYER H ; HASHIMOTO Y ; SHUDO K ; KAGECHIKA H.** Regulation of retinoidal actions by diazepinylbenzoic acids. Retinoid synergists which activate the RXR-RAR heterodimers. *J Med Chem.,* 19 December 1997, vol. 40 (26), 4222-34
- **VILA, M. ; PRZEDBORSKI, S.** Genetic clues to the pathogenesis of Parkinson's disease. *Nat. Med.,* 2004, vol. 10, S58-S62
- **WAGNER CE ; JURUTKA PW ; MARSHALL PA ; GROY TL ; VAN DER VAART A ; ZILLER JW ; FURMICK JK ; GRAEBER ME ; MATRO E ; MIGUEL BV.** Modeling, synthesis and biological evaluation of potential retinoid X receptor (RXR) selective agonists: novel analogues of 4-[1-(3,5,5,8,8-pentamethyl-5,6,7,8-tetrahydro-2-naphthyl)ethynyl]benzoic acid (bexarotene. *J Med Chem.,* 08 October 2009, vol. 52 (19), 5950-66
- **WALLEN-MACKENZIE, A. ; A. MATA DE URQUIZA et al.** Nurr1-RXR heterodimers mediate RXR ligand-induced signaling in neuronal cells. *Genes Dev,* 2003, vol. 17 (24), 3036-47
- **ZETTERSTROM, R. H. ; L. SOLOMIN et al.** Dopamine neuronagenesis in Nurr1-deficient mice. *Science,* 1997, vol. 276 (5310), 248-50